# EUROPEAN PATENT APPLICATION

(11) **EP 4 545 527 A1**
(43) Date of publication of application: **30.04.2025**
(21) Application number: 23826570.6
(22) Date of filing: 21.06.2023
(51) Int. Cl.: C07D 417/14, C07D 513/04, C07D 487/04, C07D 471/04, A61K 31/4725, A61P 35/00

(54) **ESTER COMPOUNDS AND USE THEREOF**

(30) Priority: 24.06.2022 CN 202210725710
(71) Applicant: Nanjing Reju Therapeutics, Inc., Nanjing, Jiangsu 211199 (CN)
(72) Inventor: JI, Yin, Nanjing, Jiangsu 211199 (CN); WANG, Xueping, Nanjing, Jiangsu 211199 (CN); ZHAI, Hui, Nanjing, Jiangsu 211199 (CN)
(74) Representative: Calysta NV
(86) International application number: PCT/CN2023/101960
(87) International publication number: WO 2023/246925

(57) **Abstract**

Disclosed in the present invention are compounds or pharmaceutically acceptable salts, solvates, hydrates, polymorphs, co-crystals, tautomers, stereoisomers or isotope labeled compounds thereof. The compounds are as shown in formula (I). Also disclosed in the present invention is the use of the compounds in preparation of a drug for preventing or treating diseases related to aging.

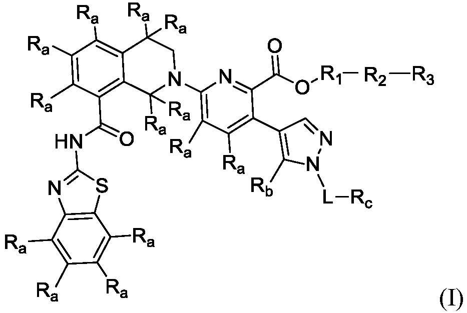

## Description

### TECHNICAL FIELD

The present invention relates to the field of medicine, in particularly to a compound capable of targeted killing senescent cells, and its use in preventing or treating diseases related to aging.

### BACKGROUND

As individuals age, the body's biological functions gradually decline, and the aging process of individual aging is often accompanied by the onset of various age-related diseases. Consequently, developing effective intervention strategies for aging is crucial for prolonging the healthy lifespan of the elderly and reducing the incidence of age-related diseases. The accumulation of senescent cells in the body serves as a significant driving force behind individual aging and the emergency of age-related diseases: On the one hand, the senescence of certain proliferating cells or stem cells can lead to a decline in an individual's regeneration ability, adversely affecting the body's recovery ability and normal functions; More importantly, the senescent cells secrete a substantial amount of inflammatory factors, known as the Senescence-Associated Secretory Phenotype (SASP), which create a chronic inflammatory microenvironment that accelerates the aging process , and promotes the development of age-related diseases. Research have shown that the specific elimination of senescent cells through genetic methods or small molecules can reduce chronic inflammation, improve tissue repair capabilities, alleviate symptoms of age-related diseases such as osteoarthritis and idiopathic pulmonary fibrosis, and alleviate the decline in physiological functions in aging organisms. Therefore, the selective elimination of senescent cells represents a promising approach for treating age-related diseases and improving the physiological functions of elderly individuals.

Senescent cells exhibit several characteristics, with permanent cell cycle arrest being a common feature among all senescent cells and are mainly dependent on p16 and p21. Another broad-spectrum feature is the increased β-gal activity in senescent cells, i.e., the enzyme activity can be detected even in suboptimal conditions for enzyme activity at pH 6.0. This phenomenon is referred to as Senescence associated-β-galactosidase (SA-β-gal). Senescent cells cultured *in vitro* typically display enlarged nuclei and cytoplasm, along with a flattened cell morphology. Additional markers of senescence include the activation of the DNA damage response, elevated levels of endoplasmic reticulum stress and ROS, and enhanced secretion of the SASP. Furthermore, anti-apoptotic factors are significantly induced in senescent cells to resist apoptosis, notably through the increased expression of anti-apoptotic proteins such as BCL2/BCL-XL. Therefore, senescent cells are often referred to as are aged yet do not undergo cell death. While the induction of cellular senescence initially produced beneficial physiological effects, such as inhibiting tumor formation and promoting wound healing, it has been subsequently discovered that the long-term presence of senescent cells in tissues may contribute to the aging and the development of age-related diseases, Moreover inflammatory factors secreted by the senescent cells may further promote tumorigenesis.

The causal relationship between the accumulation of senescent cells and aging, as well as the onset of age-related diseases, has been clearly addressed through genetic approaches. In 2011, Professor Deursen's research team at Mayo Clinic constructed p16 promoter-driven INK-ATTAC transgenic mouse model, which selectively expresses the FKBP-CASP8 fusion protein in p16-positive senescent cells. Upon treatment with the small molecule AP20187, the FKBP-CASP8 fusion protein dimerized and induced apoptosis in p16-positive senescent cells. Significant improvements in sarcopenia, cataracts and lipodystrophy can be observed in a Bub1b-mutant premature aging mouse, where the senescent cells were intermittently removed using this method. In a 2016 study, researchers eliminated senescent cells by administering AP20187 to the naturally aged INK-ATTAC mouse every two weeks. The results showed that the elimination of p16-positive senescent cell mitigated age-related degeneration in various tissues and organs (including the kidney, heart, and adipose) and prolonged median lifespan. Professor Kirland's group implanted a small number of senescent cells into recipient mice, which was sufficient to induce persistent physical dysfunction in young mice, leading to decreased functionality and increased mortality. These findings demonstrated from multiple perspectives that the elimination of senescent cells in the body can prolong lifespan and delay the onset of age-related diseases, providing a theoretical foundation for strategies aimed at targeting and eliminating senescent cells to achieve anti-aging effects and treat age-related diseases.

Currently, several small molecule compounds have been identified that can selectively eliminate senescent cells, and these small molecules are also known as "senolytics". Due to the heterogeneity of senescent cells, these senolytics can achieve selective killing of senescent cells to some extent. However, there remains significant potential for improvement in reducing toxicity and enhancing specificity of cell elimination. Early senolytics primarily targeted key components of the Senescent Cell anti-Apoptotic Pathways (SCAP), by inhibiting the anti-apoptotic signals in senescent cells, thereby inducing their death. In comparison to normal cells, the expression of the anti-apoptotic protein BCL2/BCL-XL is significantly elevated in senescent cells, which counteracts the activity of pro-apoptotic proteins such as Bax, rendering senescent cells resistant to apoptosis. As a dual inhibitor of the classical anti-apoptotic protein BCL2/BCL-XL, ABT263 (navitoclax) has been shown to specifically induce apoptosis in senescent cells.

A-1331852 is a small molecule inhibitor that specifically targets the Bcl-xL protein. In addition to demonstrating a very good killing effect on Bcl-xL-dependent cancer cells, A-1331852 also exhibits high selectivity for killing senescent cells. Although Bcl-xL can eliminate senescent cells, it also has a strong killing effect on normal cells at a certain concentration. Therefore, the safety profile for normal cells requires further improvement.

Exploring the differences between senescent and non-senescent cells is the fundamental for achieving targeted recognition and elimination of senescent cells. The inventors have discovered through their research that the activity of carboxylesterase in senescent cells is significantly higher than that in non-senescent cells. Therefore, carboxylesterase may be a potential marker for senescent cells, providing a theoretical foundation for the application of prodrug strategies based on the activation of such enzymes. Currently, there is a lack of Bcl-xL inhibitors derived from the modification of A-1331852 that can effectively and specifically induce apoptosis in senescent cells, and utilize such process for anti-aging and treatment of aging related diseases in mammals. The present invention takes advantage of the high esterase activity in senescent cells to develop novel prodrug compounds that meet these needs and provide related benefits.

### SUMMARY OF THE INVENTION

In order to address at least one of the technical challenges present in the prior art, the present invention provides a class of compounds. These compounds can selectively eliminate senescent cells and tumor cells within a broader safety margin, while minimizing the side effects associated with killing normal cells, thereby offering enhanced selectivity and safety.

A first aspect of the present application provides a compound represented by formula I, or a pharmaceutically acceptable salt, solvate, hydrate, polymorph, co-crystal, tautomer, stereoisomer, or isotope-labeled compound thereof, 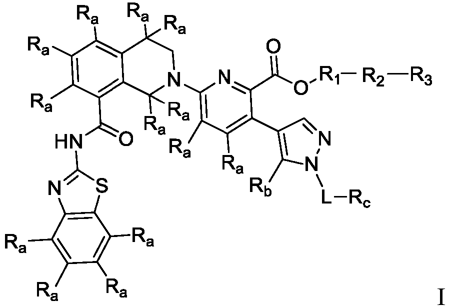 wherein,
R₁ is selected from substituted or unsubstituted C₁-C₁₀ alkylene;
R₂ is selected from the group consisting of -O-C(O)- and -O-C(O)-O-;
R₃ is selected from the group consisting of substituted or unsubstituted C1-C10 chain alkyl, substituted or unsubstituted C3-C20 cycloalkyl, substituted or unsubstituted C2-C20 chain alkenyl, substituted or unsubstituted C2-C20 chain alkynyl, substituted or unsubstituted C6-C20 aryl, substituted or unsubstituted C1-C20 heteroaryl, and substituted or unsubstituted C2-C20 heteroalicyclic group; or R₁ is absent or is selected from substituted or unsubstituted C₁-C₁₀ alkylene, R₂ is absent, and R₃ is selected from substituted or unsubstituted C2-C20 heteroalicyclic group;
each Rₐ is independently selected from the group consisting of hydrogen, halogen, substituted or unsubstituted C1-C10 alkoxy, substituted or unsubstituted C1-C10 alkyl, substituted or unsubstituted C6-C20 aryl, substituted or unsubstituted C1-C20 heteroaryl, nitro, and cyano;
R_{b} is selected from the group consisting of hydrogen, substituted or unsubstituted C1-C10 alkyl, substituted or unsubstituted C6-C20 aryl, and substituted or unsubstituted C1-C20 heteroaryl;
L is selected from substituted or unsubstituted C₁-C₁₀ alkylene; and
R_{c} is selected from substituted or unsubstituted adamantyl.

In some embodiments, the substituent involved in the substitution is selected from the group consisting of halogen atom, hydroxyl, mercapto, amino, nitro, cyano, carboxy, acyl, C1-C10 alkoxy, C6-C20 aryl, C1-C20 heteroaryl, C2-C20 heteroalicyclic group, C1-C10 alkyl, C3-C8 cycloalkyl, C2-C8 chain alkenyl or C2-C8 alkynyl, halogen and/or hydroxyl substituted C1-C10 alkyl, halogen and/or hydroxyl substituted C3-C8 cycloalkyl, halogen and/or hydroxyl substituted C2-C8 chain alkenyl, halogen and/or hydroxyl substituted C2-C8 alkynyl, and halogen and/or hydroxyl substituted C3-C8 cycloalkyl.

In some embodiments, the ring of the C2-C20 heteroalicyclic group optionally additionally contains 1 or 2 heteroatoms selected from N or O.

In some embodiments, the C2-C20 heteroalicyclic group is optionally substituted with one or more substituents selected from the group consisting of halogen atom, cyano, nitro, C6-C10 aryl, C1-C10 heteroaryl, C1-C6 chain alkoxy, C6-C10 aryloxy, C2-C10 heteroalicyclic group, amino, hydroxyl, mercapto, carbonyl, carboxyl, acyl, 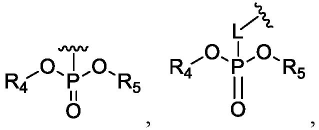 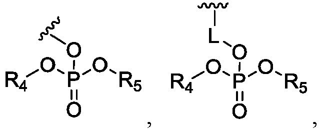 and -NR₄R₅, R₄ and R₅ are independently selected from the group consisting of hydrogen, C6-C10 aryl, C1-C10 heteroaryl, C1-C8 chain alkyl, C3-C8 cycloalkyl, C2-C8 chain alkenyl, and C2-C8 alkynyl, the aryl and heteroaryl are optionally substituted with halogen atom, hydroxyl, mercapto, amino, nitro, cyano, carboxyl, acyl, C1-C8 alkoxy, C6-C10 aryl, C1-C10 heteroaryl, C2-C10 heteroalicyclic group, C1-C8 alkyl, C3-C8 cycloalkyl, C2-C6 chain alkenyl, or C2-C8 alkynyl, optionally, wherein the substituents of at least two positions together form C3-C10 aliphatic ring, C2-C10 heteroalicyclic ring, C6-C10 aromatic ring, or C1-C10 heteroaromatic ring.

In some embodiments, the compound is represented by formula III, IV or V: 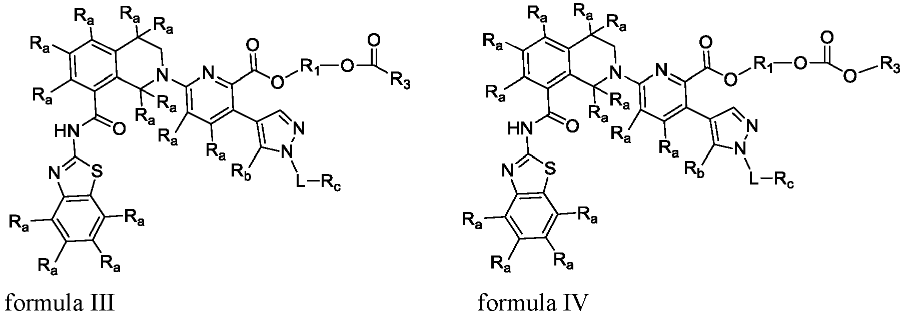 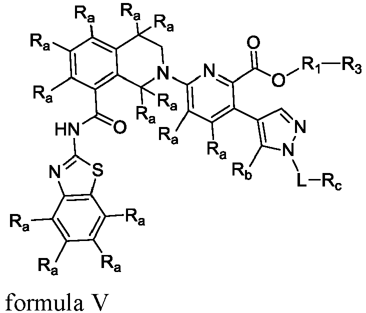
in formula III and formula IV, R₁ is independently selected from substituted or unsubstituted C₁-C₁₀ alkylene, preferably, R₁ is independently selected from substituted or unsubstituted C₁-C₆ alkylene, preferably, R₁ is independently selected from substituted or unsubstituted C₁-C₄ alkylene; and Rₐ, R_{b}, R₃, R_{c}, and L are defined as in formula I; and
in formula V, R₁ is absent or independently selected from substituted or unsubstituted C₁-C₁₀ alkylene, preferably, R₁ is independently selected from substituted or unsubstituted C₁-C₆ alkylene, preferably, R₁ is independently selected from substituted or unsubstituted C₁-C₄ alkylene; R₃ is selected from substituted or unsubstituted C2-C20 heteroalicyclic group, and Rₐ, R_{b}, R_{c}, and L are defined as in formula I.

In some embodiments, the C2-C20 heteroalicyclic group is optionally substituted with a substituent selected from the group consisting of halogen atom, hydroxyl, mercapto, amino, nitro, cyano, C1-C10 alkoxy, C1-C10 alkyl, C3-C8 cycloalkyl, C2-C8 chain alkenyl, C2-C8 alkynyl, 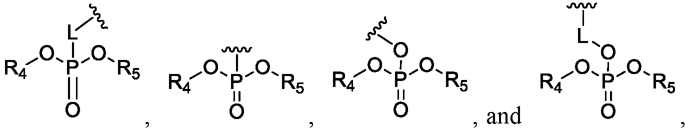 and each R₅ is independently selected from the group consisting of hydrogen and C1-C6 alkyl.

In some embodiments, the C2-C20 heteroalicyclic group is C4-C8 heteroalicyclic group; the heteroalicyclic group is optionally substituted with halogen, -NH₂, -OH, -NO₂, carbonyl, - CH₂OH, carboxyl, methyl, ethyl, propyl, isopropyl, methoxy, ethoxy, propoxy, or isopropoxy.

In some embodiments, the C2-C20 heteroalicyclic group is selected from a group represented by the following groups: 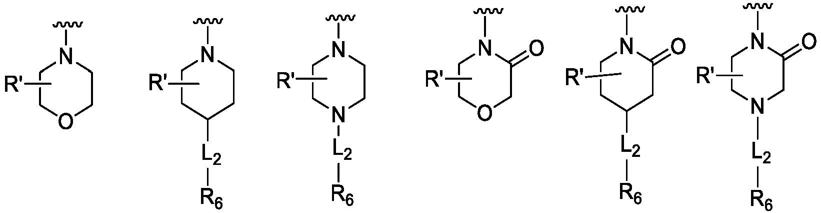 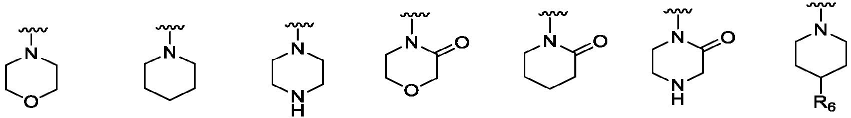 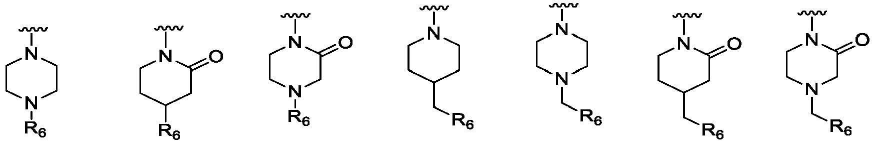 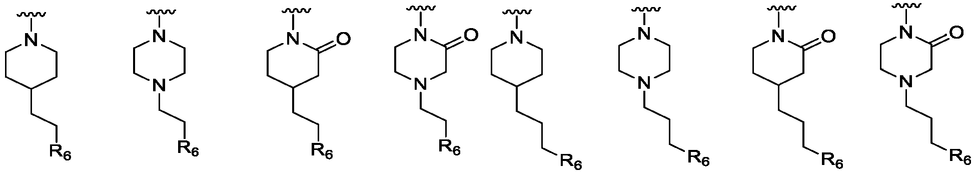 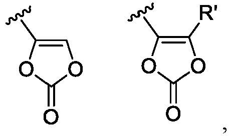
R' each independently represents no substituent, single substituent, or multiple substituents, and each substituent is independently selected from the group consisting of deuterium, hydroxyl, halogen, NH₂, carboxyl (-COOH), 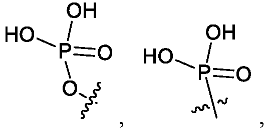 C1-C6 chain alkyl, halogen-substituted C1-C6 chain alkyl, hydroxyl-substituted C1-C6 chain alkyl, amino-substituted C1-C6 chain alkyl, morpholine-substituted C1-C6 chain alkyl, -COO-C1-C6 chain alkyl, cyano, C1-C6 chain alkoxy, C3-C6 cycloalkyl, halogen-substituted C3-C6 cycloalkyl, hydroxyl-substituted C3-C6 cycloalkyl, phenyl, and benzyl;
L₂ is absent or C1-C6 alkylene, halogen, hydroxyl, or C1-C6 alkoxy-substituted C1-C6 alkylene, preferably methylene, ethylene, or propylene;
R₆ is H, deuterium, halogen, hydroxyl, NH₂, carboxyl (-COOH), -CONH₂, sulfonic acid group (-SO₃H), -SO₂-C1-C6 chain alkyl, 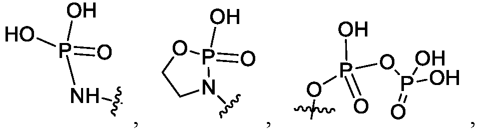 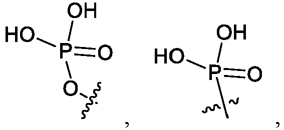 C1-C6 chain alkyl, halogen-substituted C1-C6 chain alkyl, morpholine-substituted C1-C6 chain alkyl, -COO-C1-C6 chain alkyl, cyano, C1-C6 chain alkoxy, hydroxyl-substituted C1-C6 chain alkyl, amino-substituted C1-C6 chain alkyl, C3-C6 cycloalkyl, halogen-substituted C3-C6 cycloalkyl, hydroxyl-substituted C3-C6 cycloalkyl, phenyl, or benzyl.

In some embodiments, each Rₐ is independently selected from the group consisting of hydrogen, halogen, substituted or unsubstituted C1-C10 alkoxy, and substituted or unsubstituted C1-C10 alkyl. In some embodiments, each Rₐ is independently selected from the group consisting of hydrogen, halogen, substituted or unsubstituted C1-C5 alkoxy, and substituted or unsubstituted C1-C5 alkyl.

In some embodiments, each Rₐ is hydrogen.

In some embodiments, R_{b} is selected from substituted or unsubstituted C1-C6 alkyl, preferably substituted or unsubstituted C1-C3 alkyl, and more preferably methyl.

In some embodiments, L is selected from substituted or unsubstituted C1-C6 alkylene, preferably substituted or unsubstituted C1-C3 alkylene. In some embodiments, L is methylene.

In some embodiments, the compound is represented by formula II: 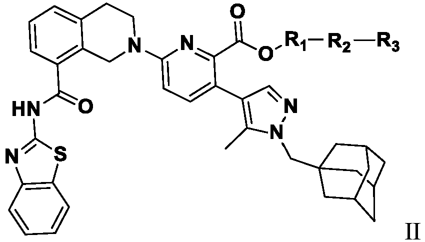 in formula II, R₁, R₂, and R₃ are defined as in formula I, that is:
R₁ is selected from C₁-C₁₀ alkylene;
R₂ is selected from the group consisting of -O-C(O)- and -O-C(O)-O-;
R₃ is selected from the group consisting of substituted or unsubstituted C1-C10 chain alkyl, substituted or unsubstituted C3-C20 cycloalkyl, substituted or unsubstituted C2-C20 chain alkenyl, substituted or unsubstituted C2-C20 chain alkynyl, substituted or unsubstituted C6-C20 aryl, substituted or unsubstituted C1-C20 heteroaryl, and substituted or unsubstituted C2-C20 heteroalicyclic group.

In some embodiments, R₁ is substituted or unsubstituted C1-C6 alkylene, preferably substituted or unsubstituted C1-C3 alkylene, more preferably methylene.

In some embodiments, R₃ is selected from the group consisting of substituted or unsubstituted C1-C8 chain alkyl, substituted or unsubstituted C3-C10 cycloalkyl, substituted or unsubstituted C2-C8 chain alkenyl, substituted or unsubstituted C2-C8 chain alkynyl, substituted or unsubstituted C6-C12 aryl, substituted or unsubstituted C2-C12 heteroaryl, and substituted or unsubstituted C1-C12 heteroalicyclic group.

Preferably, R₃ is selected from the group consisting of substituted or unsubstituted C1-C6 alkyl, substituted or unsubstituted C6-C12 aryl, substituted or unsubstituted C1-C10 heteroaryl, substituted or unsubstituted C3-C8 cycloalkyl, and substituted or unsubstituted C1-C10 heteroalicyclic group.

In the present application, the term "substituted or unsubstituted" refers to that one or more hydrogen atoms in the group described by it can be replaced by a substituent, and the substituent can be selected from the group consisting of halogen atom, cyano, nitro, C6-C20 aryl, C1-C20 heteroaryl, C1-C10 chain alkyl, C1-C10 chain alkoxy, C6-C20 aryloxy, C1-C20 heteroalicyclic group preferably C1-C10 heteroalicyclic group, amino, hydroxyl, mercapto, phosphate group, -OC(O)R₆, -ONR₆R₇, and -NR₆R₇, R₆ and R₇ are independently selected from the group consisting of hydrogen, C6-C20 aryl, C1-C20 heteroaryl, C1-C8 chain alkyl, C3-C8 cycloalkyl, C2-C8 chain alkenyl, and C2-C8 chain alkynyl. The aryl and heteroaryl are optionally substituted with halogen atom, hydroxyl, mercapto, amino, nitro, cyano, carboxyl, acyl, C1-C6 alkoxy, C6-C20 aryl, C1-C20 heteroaryl, C2-C20 heteroalicyclic group, C1-C10 alkyl, C3-C8 cycloalkyl, C2-C8 chain alkenyl, or C2-C8 chain alkynyl, wherein optionally the substituents of at least two positions together form aliphatic ring such as C3-C20 aliphatic ring, heteroalicyclic ring such as C2-C20 heteroaliphatic ring, aromatic ring such as C6-C20 aromatic ring, or heteroaromatic ring such as C1-C20 heteroaromatic ring.

In some embodiments, R₃ is selected from the following groups:

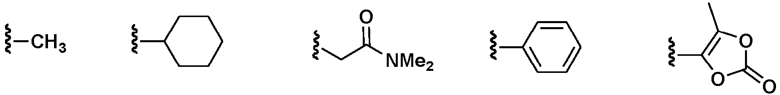

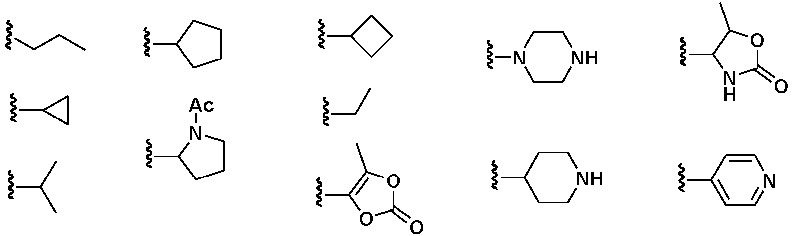

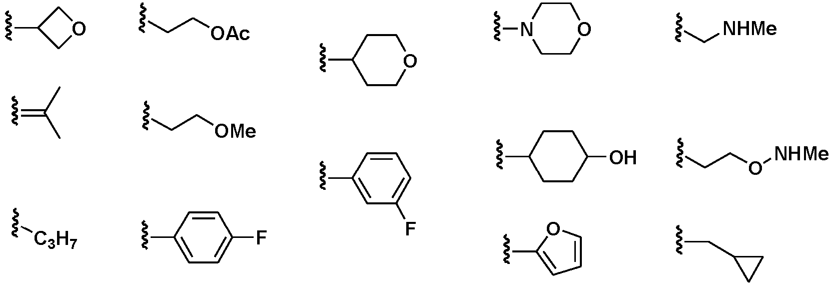

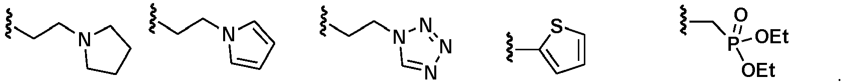

In some embodiments, the compound is selected from the following compounds:

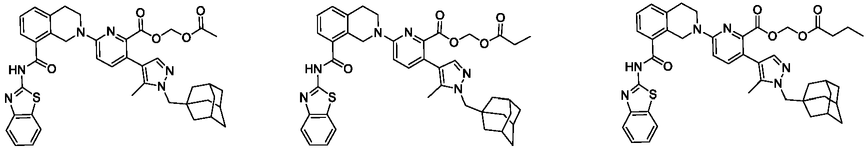

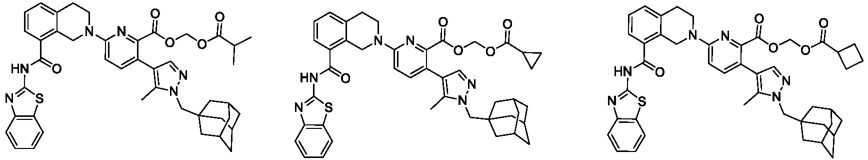

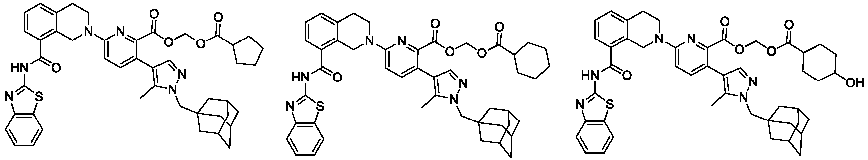

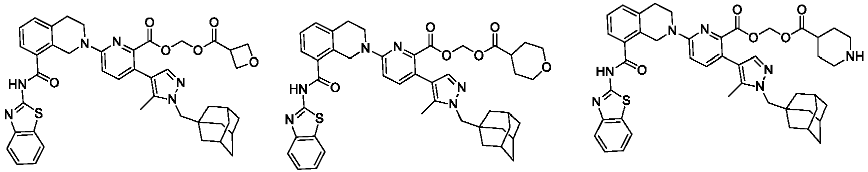

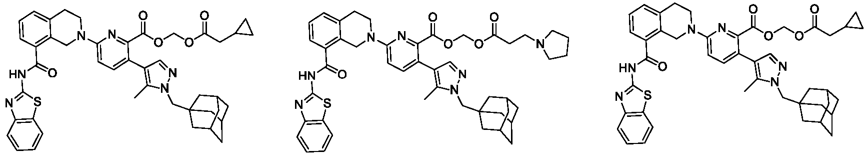

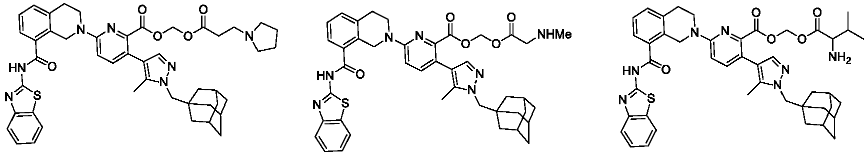

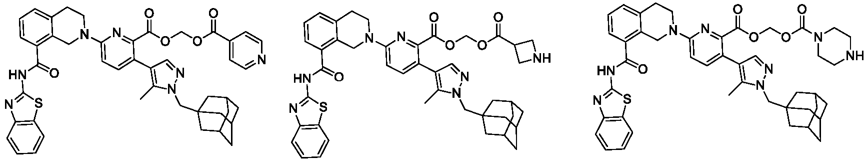

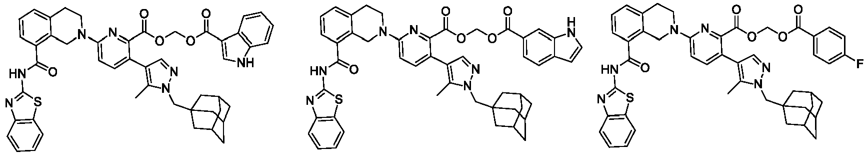

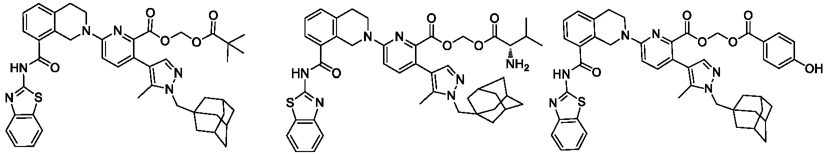

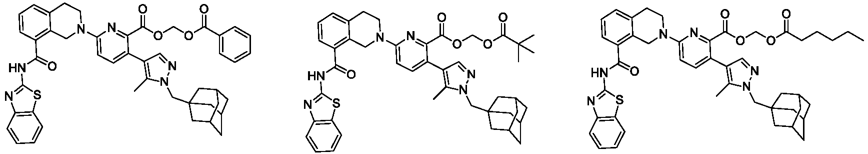

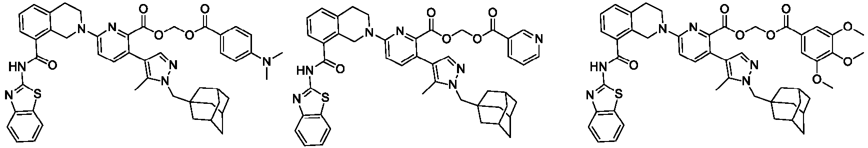

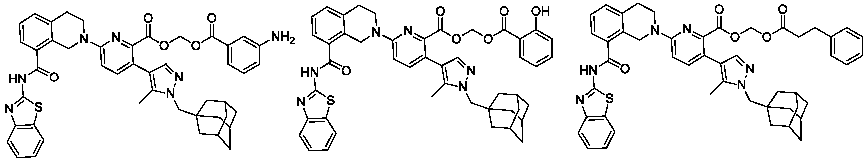

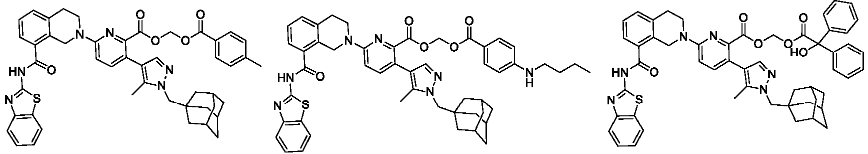

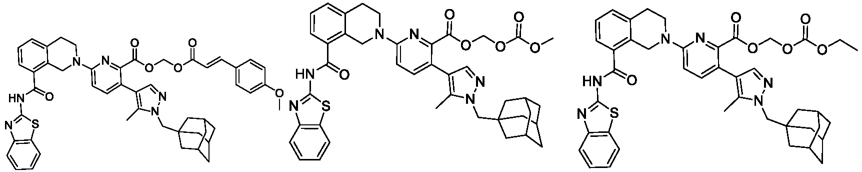

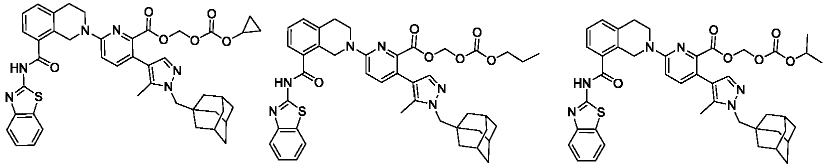

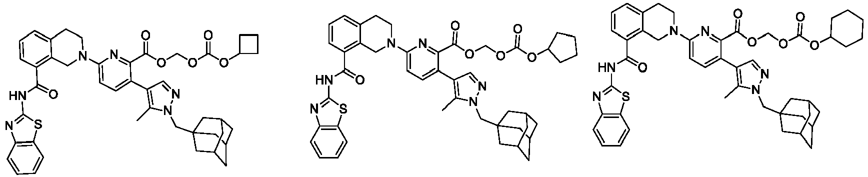

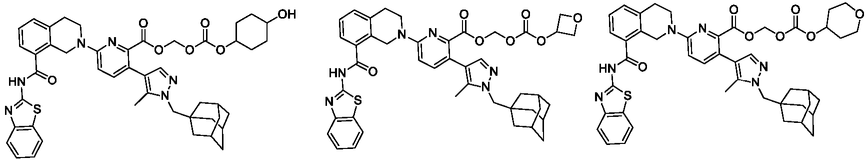

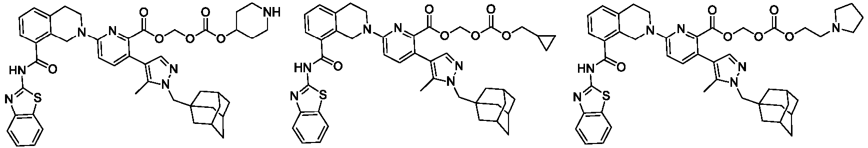

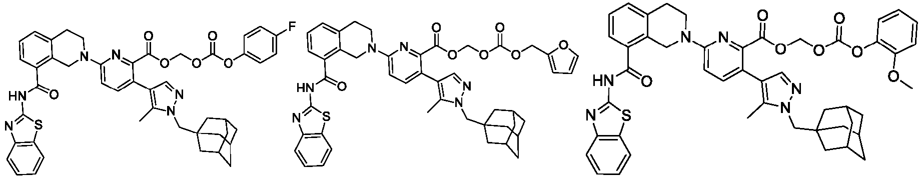

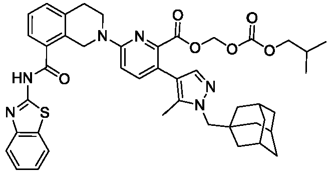

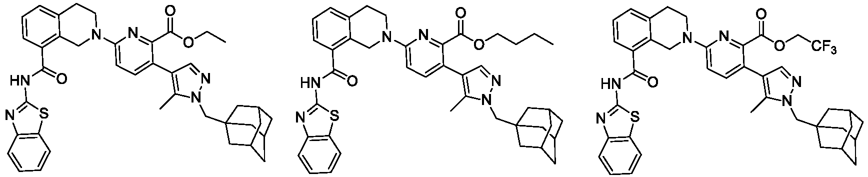

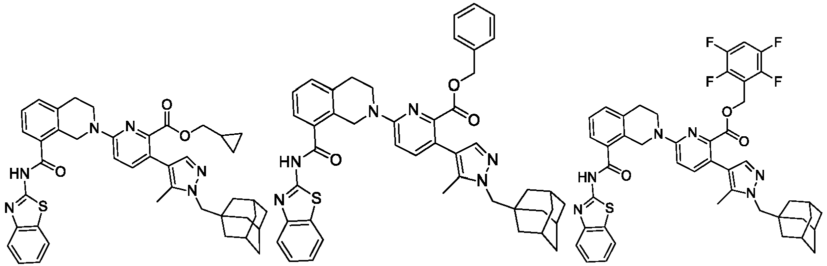

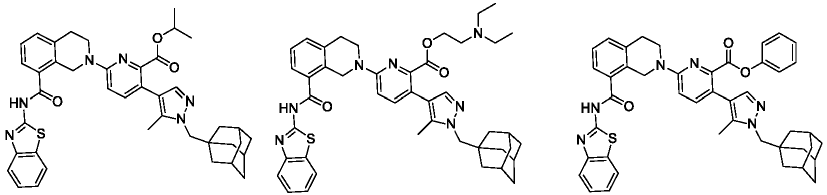

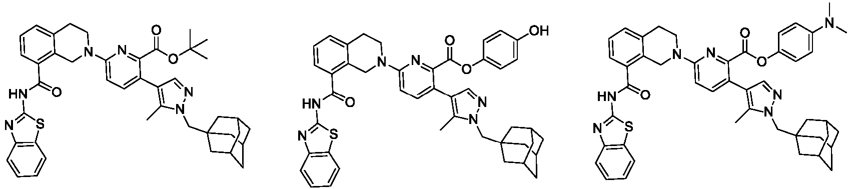

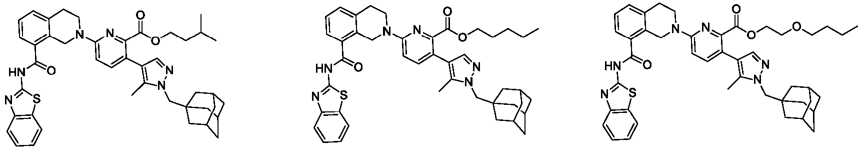

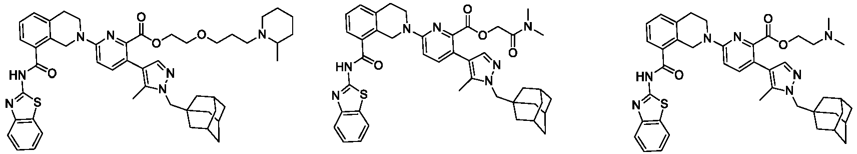

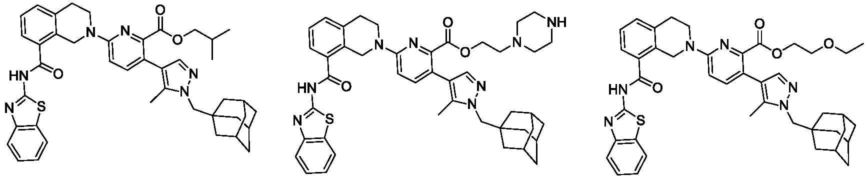

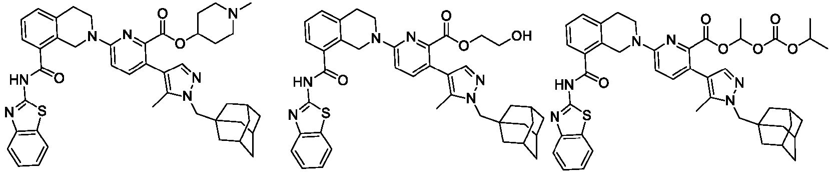

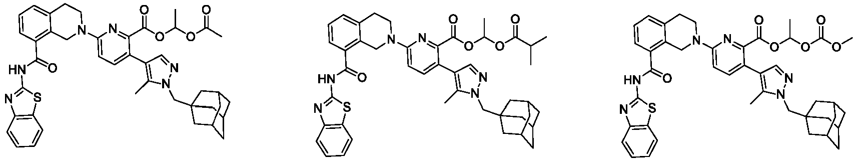

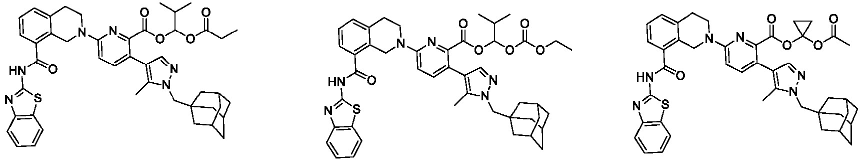

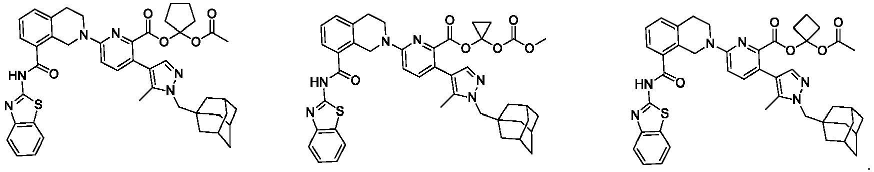

A second aspect of the present application provides a method for preventing or treating age-related diseases. The method comprises administering to a subject in need a therapeutically effective dosage of the compound or a pharmaceutically acceptable salt, solvate, hydrate, polymorph, co-crystal, tautomer, stereoisomer or isotope labeled compound thereof in the first aspect.

Specifically, The diseases associated with aging are primarily characterized by the accumulation of senescent cells, and preferably the disease is one or more selected from the group consisting of idiopathic pulmonary fibrosis, pulmonary fibrosis, hepatic fibrosis, renal fibrosis, inflammation and tissue fibrosis and atrophy of upper respiratory tract and lungs caused by viruses, cystic fibrosis, myelofibrosis, myocardial fibrosis, cutaneous fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritis neuropathy, keratitis sicca, Fuch's corneal dystrophy, presbyopia, cataract, degenerative vitreous disorder including vitreomacular traction syndrome, macular hole, retinal tear, retinal detachment, and proliferative vitreoretinopathy, osteoarthritis, disc herniation, osteoporosis, Alzheimer's disease, Parkinson's disease, atherosclerosis, chronic obstructive pulmonary disease, diabetes, diabetic nephropathy, scar, superficial scar or flat scars, rope scar or contracted scar, webbed scar, depressed scar, atrophic scar, bridge scar and pedunculated scar, hypertrophic scar, keloid, scar cancer, scleroderma, morphea, linear scleroderma, guttate scleroderma, acroscleroderma, diffuse scleroderma, CREST syndrome, acute coronary syndrome, myocardial infarction, stroke, hypertension, obesity, lipodystrophy, coronary artery disease, cerebrovascular disease, periodontal disease, cancer treatment-related disabilities such as atrophy and fibrosis in various tissues, brain and heart damage and treatment-related myelodysplastic syndrome, promyelocytic syndrome, ataxia telangiectasia, Fanconi anemia, Friedreich's ataxia, congenital dyskeratosis, aplastic anemia, aneurysm, inflammatory bowel disease, lipoatrophy, renal transplant failure, sarcopenia, wound healing, alopecia, cardiomyocyte hypertrophy, glomerulosclerosis, and cancer.

A second aspect of the present application provides use of a compound or a pharmaceutically acceptable salt, solvate, hydrate, polymorph, co-crystal, tautomer, stereoisomer, or isotope labeled compound thereof in the preparation of a medicament for the prevention or treatment of diseases related to aging, wherein said compound is the compound in the first aspect of the present application.

Specifically, The diseases associated with aging are primarily characterized by the accumulation of senescent cells, and preferably the disease is one or more selected from the group consisting of idiopathic pulmonary fibrosis, pulmonary fibrosis, hepatic fibrosis, renal fibrosis, inflammation and tissue fibrosis and atrophy of upper respiratory tract and lungs caused by viruses, cystic fibrosis, myelofibrosis, myocardial fibrosis, cutaneous fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritis neuropathy, keratitis sicca, Fuch's corneal dystrophy, presbyopia, cataract, degenerative vitreous disorder, including vitreomacular traction syndrome, macular hole, retinal tear, retinal detachment, and proliferative vitreoretinopathy, osteoarthritis, disc herniation, osteoporosis, Alzheimer's disease, Parkinson's disease, atherosclerosis, chronic obstructive pulmonary disease, diabetes, diabetic nephropathy, scar, superficial scar or flat scars, rope scar or contracted scar, webbed scar, depressed scar, atrophic scar, bridge scar and pedunculated scar, hypertrophic scar, keloid, scar cancer, scleroderma, morphea, linear scleroderma, guttate scleroderma, acroscleroderma, diffuse scleroderma, CREST syndrome, acute coronary syndrome, myocardial infarction, stroke, hypertension, obesity, lipodystrophy, coronary artery disease, cerebrovascular disease, periodontal disease, cancer treatment-related disabilities such as atrophy and fibrosis in various tissues, brain and heart damage and treatment-related myelodysplastic syndrome, promyelocytic syndrome, ataxia telangiectasia, Fanconi anemia, Friedreich's ataxia, congenital dyskeratosis, aplastic anemia, aneurysm, inflammatory bowel disease, lipoatrophy, renal transplant failure, sarcopenia, wound healing, alopecia, cardiomyocyte hypertrophy, glomerulosclerosis, and cancer.

The compound provided by the present application can reduce the side effects associated with the killing of normal cells and selectively kill senescent cells and tumor cells within a wider safety window, thereby offering enhanced selectivity and safety.

### BRIEF DESCRIPTION OF DRAWINGS

Figure 1 shows the results of carboxylesterase activity in normal and senescent cells.
Figure 2 shows the results of cell viability of the compounds on normal and senescent cells.
Figure 3 shows that intravitreal (IVT) administration of RZ001-RZ006 and RZ201 resulted in statistically significant improvements in avascular zone and angiogenesis.
Figure 4 shows that intravitreal (IVT) administration of RZ001, RZ002, and RZ201 in a diabetic macular model resulted in improved vascular permeability in both the retinal and choroidal vascular leakage at this dose level.
Figure 5 shows that intravitreal (IVT) administration of RZ001, RZ002, and RZ201 resulted in statistically significant improvements in fluorescence intensity indicators in a CNV macular model.
Figure 6 shows that administration of RZ001, RZ002, and RZ201 via intraperitoneal (IP) injection resulted in statistically significant improvements in WBC, HYP and pathology scores in a pulmonary fibrosis (IPF) model.
Figure 7 shows that administration of RZ001, RZ002, and RZ201 via intra-articular injection resulted in statistically significant improvements in plantar mechanical pain threshold and pathology scores of safranin fast green staining in an osteoarthritis (OA) model.
Figure 8 shows that administration of RZ001, RZ002, and RZ201 via intraperitoneal (IP) injection resulted in statistically significant improvements in Aβ42 pathogenic protein indicators in an Alzheimer's disease (AD) mouse model.
Figure 9 shows that administration of RZ001, RZ002, and RZ201 via intraperitoneal (IP) injection resulted in statistically significant improvements in serum aspartate aminotransferase (AST) and alanine aminotransferase (ALT) levels and pathology scores in a liver fibrosis model.
Figure 10 shows that administration of RZ001, RZ002, and RZ201 by applying resulted in statistically significant improvements in epidermal thickness scores of the skin in a naturally aging mouse model.
Figure 11 shows that administration of RZ001, RZ002, and RZ201 via intraperitoneal (IP) injection resulted in statistically significant improvements in serum creatinine (Cr) and urea nitrogen (BUN) levels and pathology in a renal fibrosis model.
Figure 12 shows that administration of RZ001 resulted in a statistically significant improvement in scar hyperplasia index in a rabbit ear hypertrophic scar model.

### DETAILED DESCRIPTION OF THE INVENTION

The technical solutions of the present invention will be described in detail in combination with the following examples and figures: the examples are carried out on the premise that the present invention is a technical solution, and detailed embodiments and processes are provided, but the embodiments provided herein are exemplary and are intended to be used for explaining the present invention, and are not to be construed as a limitation of the present invention. The conditions and methods not specified in the following examples are carried out conventionally.

### Definition

The term "alkyl" refers to an aliphatic hydrocarbon group, which may be branched or linear alkyl. Depending on the structure, the alkyl can be monovalent group or bivalent group (i.e., alkylene). In the present invention, the alkyl is preferably an alkyl having 1 to 8 carbon atoms, more preferably a "lower alkyl" having 1 to 6 carbon atoms, and even more preferably an alkyl having 1 to 4 carbon atoms. Typical alkyl includes, but is not limited to, methyl, ethyl, propyl, butyl, pentyl, hexyl, and the like. It should be understood that, the "alkyl" as used herein includes all possible configurations and conformations of the alkyl. For example, the "propyl" mentioned herein includes n-propyl and isopropyl, the "butyl" includes n-butyl, isobutyl, and tert-butyl, and the "pentyl" includes n-pentyl, isopentyl, neopentyl, tert-pentyl, and pentyl-3-yl, and the like.

The term "alkoxy" refers to -O-alkyl, wherein the alkyl is as defined herein. Typical alkoxy includes, but is not limited to, methoxy, ethoxy, propoxy, butoxy, pentyloxy, hexyloxy, and the like.

The term "cycloalkyl" refers to monocyclic or polycyclic group containing only carbon and hydrogen. Cycloalkyl includes a group having 3 to 12 ring atoms. Depending on the structure, a cycloalkyl can be monovalent group or bivalent group (e.g., cycloalkylene). In the present invention, the cycloalkyl is preferably a cycloalkyl having 3 to 8 carbon atoms, and more preferably a "lower cycloalkyl" having 3 to 6 carbon atoms. Examples of cycloalkyl include, but are not limited to, cyclopropyl, cyclobutyl, cyclopentyl, cyclohexyl, cycloheptyl, cyclooctyl, cyclopentenyl, cyclohexenyl, cycloheptenyl, and adamantyl.

The term "aryl" refers to an aromatic ring in which each of the atoms making up the ring is a carbon atom. Aromatic ring can be composed of five, six, seven, eight, nine, or more than nine atoms. Aryl may be optionally substituted. Examples of aryl include, but are not limited to, phenyl, naphthyl, phenanthryl, anthracenyl, fluorenyl, and indenyl. Depending on the structure, an aryl may be monovalent group or bivalent group (i.e., arylene).

The term "heteroaryl" refers to an aromatic group containing one or more ring heteroatoms selected from the group consisting of nitrogen, oxygen, and sulfur. The N-containing "heteroaryl" moiety refers to an aromatic group in which at least one skeleton atom in the ring is a nitrogen atom. Depending on the structure, a heteroaryl can be monovalent group or bivalent group (i.e., heteroarylene). Examples of heteroaryl include, but are not limited to, pyridyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolyl, isoquinolyl, indolyl, benzimidazolyl, benzofuranyl, indazolyl, indolizinyl, phthalazinyl, pyridazinyl, isoindolyl, pteridinyl, purinyl, oxadiazolyl, thiadiazolyl, furazanyl, benzofurazanyl, benzothienyl, benzothiazolyl, benzoxazolyl, quinazolinyl, naphthyridinyl, furopyridyl, and the like.

The term "alicyclic group" or "cycloalkyl" used herein refers to a non-aromatic ring formed by three or more carbon atoms, wherein the bond between two adjacent carbon atoms in the ring can be single bonds, double bonds or triple bonds. The number of rings can be one or more. Non-limiting examples include cyclopropyl, cyclobutyl, cyclopentyl, cyclohexyl, cycloheptyl, cyclohexenyl, cyclopentenyl, cyclohexadienyl, and the like.

The term "heterocycloalkyl" or "heteroalicyclic group" used herein refers to a non-aromatic ring in which one or more atoms constituting the ring are heteroatoms selected from the group consisting of nitrogen, oxygen and sulfur. Heterocycloalkyl may be composed of three, four, five, six, seven, eight, nine or more than nine atoms. Heterocycloalkyl may be optionally substituted. Examples of heteroalicyclic group include, but are not limited to, lactam, lactone, cyclicimine, cyclicthioimine, cyclic carbamate, tetrahydrothiopyran, 4H-pyran, tetrahydropyran, piperidine, 1,3-dioxin, 1,3-dioxane, 1,4-dioxin, 1,4-dioxane, piperazine, 1,3-oxathiane, 1,4-oxathiine, 1,4-oxathiane, tetrahydro-1,4-thiazine, 2H-1,2-oxazine, maleimide, succinimide, barbituric acid, thiobarbituric acid, dioxopiperazine, hydantoin, dihydrouracil, morpholine, trioxane, hexahydro-1,3,5-triazine, tetrahydrothiophene, tetrahydrofuran, pyrroline, pyrrolidine, imidazolidine, pyrrolidone, pyrazoline, pyrazolidine, imidazoline, imidazolidine, 1,3-dioxolene, 1,3-dioxolane, 1,3-dithiolene, 1,3-dithiolane, isoxazoline, isoxazolidine, oxazoline, oxazolidine, oxazolidinone, thiazoline, thiazolidine and 1,3-oxathiolane. Depending on the structure, the heteroalicyclic group may be monovalent group or bivalent group (i.e., heterocycloalkylene).

The term "halo" or "halogen" used herein refers to fluorine, chlorine, bromine, and iodine.

The term "carbonyl" used herein refers to an organic functional group (C=O) formed by connecting carbon and oxygen, through a double bond.

The term "optional" used herein refers to that one or more of subsequent events may occur or may not occur, and includes both events that occur and events that do not occur.

The salt formed by the compound in the present invention are also within the scope of the present invention. Unless otherwise specified, the compound in the present invention is understood as including its salts. The term "salt" used herein refers to an acidic or basic salt formed from inorganic or organic acid and base. Furthermore, when the compound of the present invention contains a basic fragment, it includes but is not limited to pyridine or imidazole, and when it contains an acidic fragment, it includes but is not limited to carboxylic acid, and the zwitterion ("inner salt") that may be formed is included in the scope of the term "salt". Pharmaceutically acceptable (i.e., nontoxic and physiologically acceptable) salts are preferred, although other salts are also useful, for example, in isolation or purification steps during preparation. The compounds of the present invention may form salts, for example, obtained from the reaction of the compound I with a certain amount of, for example, an equivalent amount of acid or base, salting out in a medium, or freeze-drying in an aqueous solution.

The basic fragments contained in the compound of the present invention include but are not limited to amines, pyridine or imidazole ring, which may form salt with organic or inorganic acid. Typical acids that can form salts include acetates (e.g., acetic acid or trihaloacetic acid, e.g. trifluoroacetic acid), adipate, alginate, ascorbate, aspartate, benzoate, benzenesulfonate, hydrosulfate, borate, butyrate, citrate, camphorate, camphorsulfonate, cyclopentanepropionate, diglycolate, dodecyl sulfate, ethanesulfonate, fumarate, gluceptate, glycerophosphate, hemisulfate, heptylate, hexanoate, hydrochloride, hydrobromide, hydroiodide, hydroxy-ethanesulfonate (e.g., 2-hydroxy-ethanesulfonate), lactate, maleate, methanesulfonate, naphthalenesulfonate (e.g., 2-naphthalenesulfonate), niacinate, nitrate, oxalate, pectinate, persulfate, phenylpropionate (e.g., 3-phenylpropionate), phosphate, picrate, neopentanoate, propionate, salicylate, succinate, sulfate (e.g., formed with sulfuric acid), sulfonate, tartrate, thiocyanate, toluenesulfonate such as p-toluenesulfonate, dodecanoate, and the like.

The acidic fragments contained in the compound of the present invention include but are not limited to carboxylic acid, which may form salt with various organic or inorganic bases. Typical salts formed with base include ammonium salt, alkali metal salts such as sodium, lithium, and potassium salts, alkaline-earth metal salts such as calcium and magnesium salts, and salts formed with organic base (such as organic amine), such as benzathine, dicyclohexylamine, hypamine (salt formed with *N,N*-bis(dehydroabietyl)ethylenediamine), *N-*methyl-D-glucamine, N-methyl-D-glucamide, tert-butylamine, and salts formed with amino acids such as arginine and lysine, and the like. Basic nitrogen-containing group can be combined with halide quaternary ammonium salts, such as small molecule alkyl halide (e.g. chloride, bromide and iodide of methyl, ethyl, propyl and butyl), dialkyl sulfate (e.g. dimethyl sulfate, diethyl sulfate, dibutyl sulfate and dipentyl sulfate), long-chain halide (e.g. chloride, bromides and iodide of decyl, dodecyl, tetradecyl and tetradecyl), aralkyl halide (e.g. benzyl and phenyl bromides), and the like.

Prodrug and solvate of the compounds of the present invention are also within the scope of the present invention. The term "prodrug" used herein refers to a compound that can be converted to produce the compound, salt, or solvate of the present invention undergoing chemical transformation by metabolic or chemical processes when treating related diseases. "Solvate" refers to a solvent addition form containing a stoichiometric or non-stoichiometric amount of solvent. Some compounds tend to trap a fixed molar ratio of solvent molecules in the crystalline solid state, thereby forming a solvate. If the solvent is water, the solvate formed is a hydrate; if the solvent is alcohol, the solvate formed is an alcoholate. A hydrate is formed by the combination of one or more water molecules with a molecule of substance, in which the water retains its molecular state H₂O. Non-limiting examples of solvate include ethanol solvate, acetone solvate, and the like.

The compound, salt or solvate of the present invention may exist in tautomeric forms (e.g. amide and imine ether). All such tautomers are parts of the present invention.

All stereoisomers of the compounds (e.g., those that may exist as asymmetric carbon atoms due to various substitutions), including their enantiomeric and diastereomeric forms, fall within the spirit scope of the present invention. The independent stereoisomers of the compounds of the present invention may not be coexist with other isomers (e.g. as a pure or substantially pure optical isomer with a special activity), or they may also be mixtures, such as racemates, or mixtures with all other stereoisomers or portions thereof. The chiral center of the present invention has S or R configuration, as defined by the recommendation of the International Union of Pure and Applied Chemistry (IUPAC) in 1974. The racemic form can be resolved by physical methods, such as stepwise crystallization, separation of crystals by derivatization into diastereomers, or separation by chiral column chromatography. Individual optical isomer can be obtained from the racemate by suitable methods, including but not limited to conventional methods, such as forming salt with an optically active acid, followed by recrystallisation.

The compounds in the present invention, which are obtained by preparation, separation, and purification in sequence, has a weight content equal to or greater than 90%, for example, equal to or greater than 95%, equal to or greater than 99% ("highly pure" compound), as listed in the description. Such "highly pure" compound of the present invention herein is also included as a part of the present invention.

All isomeric forms of the compounds of the present invention are included within the scope, including mixtures, pure form, or highly pure form. The definition of compounds in the present invention includes both cis (Z) and trans (E) isomers of alkenes, as well as cis and trans isomers of carbocyclic and heterocyclic rings.

Throughout the description, groups and substituents may be selected to provide stable fragments and compounds.

Definitions of specific functional groups and chemical terms are detailed below. For the purposes of the present invention, the chemical elements are defined in Periodic Table of the Elements, CAS version, Handbook of Chemistry and Physicsm, 75th Ed. Definitions of specific functional groups are also described therein. In addition, the basic principles of organic chemistry and specific functional groups and reactivities are described in "Organic Chemistry", Thomas Sorrell, University Science Books, Sausalito: 1999, the entire content of which is incorporated by reference.

Certain compounds of the present invention may exist in specific geometric or stereoisomeric forms. The present invention encompasses all compounds, including their cis and trans isomers, R and S enantiomers, diastereomers, (D) isomers, (L) isomers, racemic mixtures and other mixtures. In addition, asymmetric carbon atoms can represent a substituent, such as alkyl. All isomers, as well as mixtures thereof, are included in the present invention.

According to the present invention, the ratio of isomers in a mixture of isomers having same molecular formula can be varied. For example, a mixture with only two isomers can have the following combinations: 50:50, 60:40, 70:30, 80:20, 90:10, 95:5, 96:4, 97:3, 98:2, 99:1, or 100:0. All ratios of isomers are within the scope of the present invention. Similar ratios, as well as ratios for more complex mixtures of isomers that are readily understood by those skilled in the art, are also within the scope of the present invention.

The present invention also includes isotope labeled compounds that are equivalent to the original compounds disclosed herein. However. in practice, it often occurs that one or more atoms are replaced with atoms with a different atomic weight or mass number. Examples of isotopes that may be listed as compounds of the present invention include hydrogen, carbon, nitrogen, oxygen, phosphorus, sulfur, fluorine, and chlorine isotopes, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl respectively. The compound, or enantiomer, diastereomer, isomer, or pharmaceutically acceptable salt or solvate thereof in the present invention, containing the isotope or other isotopic atom of the compound specified above, are included within the scope of the present invention. Certain isotope labeled compounds of the present invention, such as radioactive isotopes of ³H and ¹⁴C, are also included, which are useful in tissue distribution experiments of drugs and substrates. Tritium, i.e. ³H, and carbon-14, i.e. ¹⁴C, are preferred for their relative ease of preparation and detection. In addition, heavier isotope substitutions such as deuterium, i.e. ²H, have advantages in certain therapies due to their good metabolic stability, such as increasing half-life or reducing dosage in the body, and therefore can be preferred in some cases. Isotope labeled compounds can be prepared using general methods by replacing non-isotopic reagents with readily available isotope labeled reagents, using the protocols disclosed in the examples.

If the synthesis of a specific enantiomer of the compound of the present invention is to be designed, it can be prepared by asymmetric synthesis, or derivatized with a chiral auxiliary, and the resulting diastereomeric mixture is separated and then the chiral auxiliary is removed to give the pure enantiomer. In addition, if the molecule contains a basic functional group, such as an amino acid, or an acidic functional group, such as carboxyl, a diastereomeric salt may be formed with a corresponding suitable optically active acid or base, and then separated by conventional means such as separation crystallization or chromatography and the like, to obtain a pure enantiomer.

As described herein, the compounds of the present invention may be substituted with any number of substituents or functional groups to expand their scope. Generally, whether the term "substituted" appears before or after the term "optionally", the general formula of the formulation of the present invention including the substituents, refers to that the hydrogen radical is replaced by a specified structural substituent. When a specific structure is substituted with multiple substituents at multiple positions, the substituents may be the same or different at each position. The term "substituted" used herein includes all permitted substitutions of organic compounds. Broadly, permitted substitutions include acyclic, cyclic, branched and unbranched, carbocyclic and heterocyclic, arylcyclic and non-arylcyclic organic compounds. In the present invention, heteroatom such as nitrogen may be supplemented with hydrogen substituents or any of permitted organic compounds described above to replenish its valence. Furthermore, the present invention is not intended to be limited in any way to the permitted substitution of organic compounds. The present invention considers that combinations of substituents and variable groups are excellent in the treatment of diseases in the form of stable compounds. The term "stable" used herein refers to a compound that is stable, and maintains the structural integrity of the compound for a sufficiently long period of detection time, preferably being effective for a sufficiently long period of time, and is used herein for the above purposes.

### Example 1. Synthesis of Compounds

### Synthesis of Compound RZ001

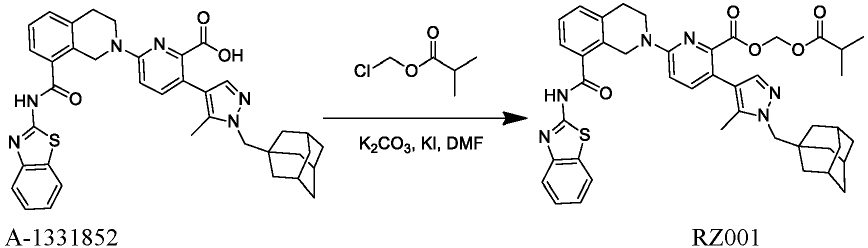

Step: To a solution of compound A-1331852 (398 mg, 0.6041 mmol, 1.00 eq) in DMF (8.0 mL), potassium carbonate (160 mg, 1.92 eq) and potassium iodide (99 mg, 0.98 eq) were added sequentially. The mixture was stirred for 10 min, then chloromethyl isobutyrate (100 mg, 1.21 eq) was added. The reaction mixture was heated to 50 °C (external oil bath temperature) and stirred for 4 h. After cooling, the reaction was quenched with water. The resultant was extracted with ethyl acetate, washed with saturated NaCl solution, dried, concentrated, and subjected to column chromatography.

¹HNMR (400 MHz, Chloroform-d) δ 7.85 (m, 1H), 7.59 (d, 1H), 7.42-7.45 (m, 2H), 7.31-7.34 (m, 4H), 7.22-7.24 (m, 1H), 6.93 (d, 1H), 5.78 (s, 2H), 5.01 (s, 2H), 4.03 (t, 2H), 3.72 (s, 2H), 3.04 (t, 2H), 2.51-2.55 (m, 1H), 2.12 (s, 3H), 1.95-2.05 (m, 3H), 1.59-1.72 (m, 12H), 1.14 (s, 3H), 1.12 (s, 3H).

### Synthesis of Compound RZ003

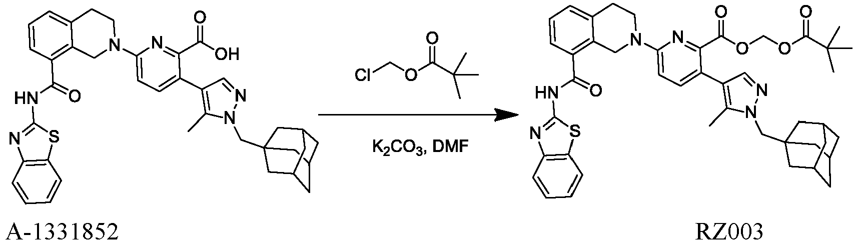

Step: To a solution of compound A-1331852 (40 mg, 0.06071 mmol, 1.00 eq) in DMF (1.0 mL), potassium carbonate (15 mg, 1.79 eq) was added. The mixture was stirred for 30 min at room temperature, followed by the addition of a solution of chloromethyl pivalate (SM7, 11 mg, 1.20 eq) in 1.0 mL DMF. The reaction mixture was heated to 50 °C (external oil bath temperature) and stirred for 5 h. The resultant was diluted with ethyl acetate, washed with water, dried, filtered, concentrated, and purified by column chromatography.

¹HNMR (400 MHz, Chloroform-d) δ 11.00 (s, 1H), 7.85 (m, 1H), 7.54 (d, 1H), 7.43 (m, 1H), 7.33 (m, 1H), 7.28-7.30(m, 4H), 7.21-7.23 (m, 1H), 6.93 (d, 1H), 5.78 (s, 2H), 5.00 (s, 2H), 4.01(t, 2H), 3.71 (s, 2H), 3.03 (t, 2H), 2.11 (s, 3H), 1.99-2.05 (m, 3H), 1.58-1.72 (m, 12H), 1.16 (s, 9H).

### Synthesis of Compound RZ004

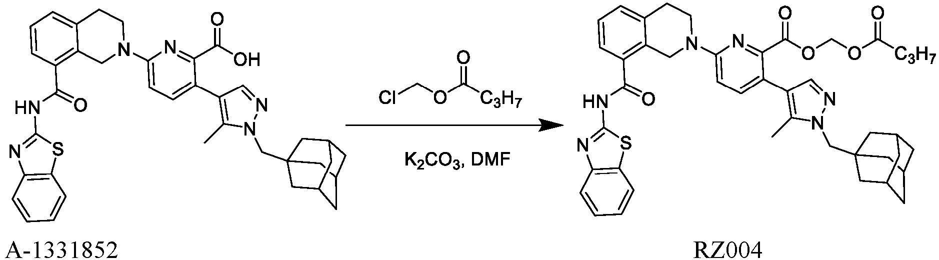

Step: To a solution of compound A-1331852 (50 mg, 0.07589 mmol, 1.00 eq) in DMF (1.0 mL), potassium carbonate (17 mg, 1.62 eq) was added. The mixture was stirred for 20 min at room temperature, followed by the addition of a solution of chloromethyl butyrate (SM8, 12 mg, 1.20 eq) in 0.8 mL DMF. The reaction mixture was heated to 50 °C (external oil bath temperature) and stirred for 4.0 h. The resultant was diluted with ethyl acetate, washed with water, dried, filtered, concentrated, and purified by column chromatography.

¹HNMR (400 MHz, Chloroform-d) δ 11.20 (s, 1H), 7.85 (m, 1H), 7.55 (d, 1H), 7.43 (d, 1H), 7.22-7.33 (m, 5H), 7.17-7.21 (m, 1H), 6.93 (d, 1H), 5.77 (s, 2H), 5.01 (s, 2H), 4.01(t, 2H), 3.71 (s, 2H), 3.03 (t, 2H), 2.28 (t, 2H), 2.10 (s, 3H), 1.94-1.99 (m, 3H), 1.57-1.72 (m, 12H), 1.24-1.33 (m, 2H), 0.92 (t, 3H).

### Synthesis of Compound RZ005

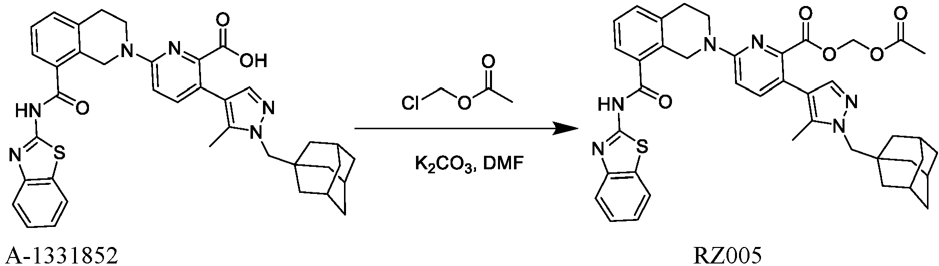

Step: To a solution of compound A-1331852 (50 mg, 0.07589 mmol, 1.00 eq) in DMF (1.0 mL), potassium carbonate (16 mg, 1.53 eq) was added. The mixture was stirred for 30 min at room temperature, followed by the addition of a solution of chloromethyl acetate (SM9, 10 mg, 1.21 eq) in 0.8 mL DMF. The reaction mixture was heated to 50 °C (external oil bath temperature) and stirred for 8 h. An additional solution of chloromethyl acetate (SM9, 8 mg, 0.97 eq) in 0.5 mL DMF was added. The reaction mixture was then heated to 50°C (external oil bath temperature) and stirred for 3 h. The resultant was diluted with ethyl acetate, washed with water, dried, filtered, concentrated, and purified by column chromatography.

¹HNMR (400 MHz, Chloroform-d) δ 7.85 (m, 1H), 7.55 (m, 2H), 7.44 (m, 1H), 7.28-7.38 (m, 5H), 6.93 (d, 1H), 5.74 (s, 2H), 5.01 (s, 2H), 4.03(t, 2H), 3.72 (s, 2H), 3.05 (t, 2H), 2.12 (s, 3H), 1.95-2.10 (m, 6H), 1.55-1.70 (m, 12H).

### Synthesis of Compound RZ012

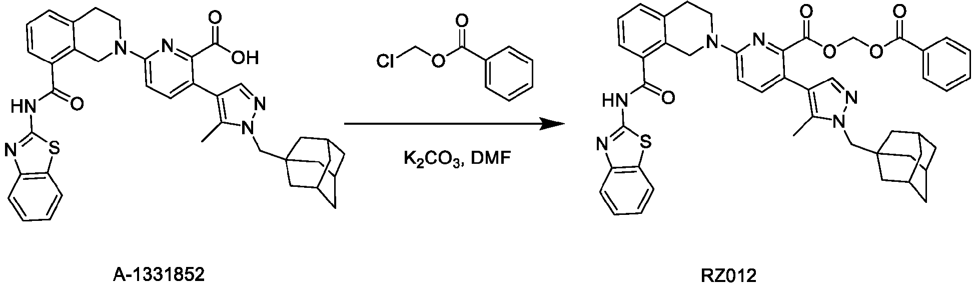

Step: To a solution of compound A-1331852 (50 mg, 0.07589 mmol, 1.00eq) in DMF (1.0 mL), potassium carbonate (16 mg, 1.53 eq) was added. The mixture was stirred for 30 min at room temperature, followed by the addition of a solution of chloromethyl benzoate (10 mg, 1.21eq) in 0.8 mL DMF. The reaction mixture was heated to 50 °C (external oil bath temperature) and stirred for 3 h. The resultant was diluted with ethyl acetate, washed with water, dried, filtered, concentrated, and purified by column chromatography.

MS(ESI) m/z = 793.5 [M+H]⁺.

According to the same method as the above examples, the following example compounds in Table 1 were prepared using commercially available compounds or by referring to the preparation methods of the intermediate compounds as indicated.

**Table 1**

| Compound ID | Structure | H NMR or MS |
|---|---|---|
| RZ007 | 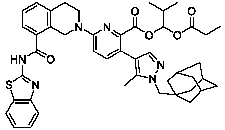 | MS(ESI) m/z = 787.1 [M+H]⁺; |
| | | ¹H NMR (400 MHz, Chloroform-d) δ 7.88 (d, J = 8.0 Hz, 1H), 7.67 (d, J = 8.0 Hz, 1H), 7.59 (d, J = 8.0 Hz, 1H), 7.48 - 7.27 (m, 6H), 7.38 (s, 2H), 6.99 (d, J = 8.8 Hz, 1H), 6.75 (d, J = 4.0 Hz, 1H), 5.37 (brs, 1H), 5.07 (d, J = 6.2 Hz, 2H), 4.05 (t, J = 6.0 Hz, 2H), 3.73 (s, 2H), 3.07 (t, J = 5.9 Hz, 2H), 2.41 - 2.20 (m, 3H), 2.14 (s, 3H), 2.04-1.98 (m, 3H), 1.73-1.62 (m,12H), 1.13 (t, J = 7.5 Hz, 3H), 0.88 (dd, J = 8.7, 6.8 Hz, 6H). |
| RZ008 | 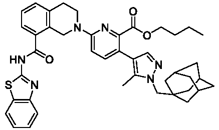 | MS(ESI) m/z = 715.5 [M+H]⁺; |
| | | ¹H NMR (400 MHz, DMSO-*d*₆) δ 8.12 (s, 1H), 7.77 (t, *J* = 8.2 Hz, 1H), 7.69 (d, *J* = 7.9 Hz, 1H), 7.61 (td, *J* = 8.0, 1.2 Hz, 2H), 7.44 (m, 1H), 7.32 (m, 1H), 7.19 (m, 1H), 7.03 - 6.90 (m, 2H), 4.45 (t, *J =* 6.4 Hz, 2H), 4.31 (d, *J =* 17.0 Hz, 1H), 4.07 (d, *J =* 17.2 Hz, 1H), 3.96 - 3.88 (m, 2H), 3.60(s, 2H), 2.90 (m, 2H), 2.71 (s, 2H), 1.97 (m, 3H), 1.78 (m, 2H), 1.61 - 1.49 (m, 13H), 1.53 - 1.37 (m, 2H), 0.92 (m, 3H). |
| RZ009 | 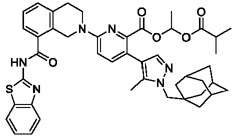 | MS(ESI) m/z = 773.5 [M+H]⁺; |
| | | ¹H NMR (400 MHz, DMSO-*d*₆) δ 8.12 (s, 1H), 7.94 (d, *J =* 5.1 Hz, 1H), 7.79 - 7.72 (m, 2H), 7.63 - 7.59 (m, 2H), 7.45 - 7.43 (m, 1H), 7.32 (m, 1H), 7.21 - 7.17 (m, 1H), 7.05 (s, 1H), 7.01 - 6.98 (m, 1H), 4.31 (d, *J =* 17.0 Hz, 1H), 4.07 (m, 2H), 3.63(s, 2H), 3.93 - 3.91 (m, 2H), 3.59 (s, 2H), 2.91 - 2.89 (m, 2H), 2.71 (s, 3H), 2.65 (d, *J =* 7.0 Hz, 1H), 1.97 (m, 3H), 1.77 (m, 3H), 1.56 - 1.50 (m, 12H), 1.23 (d, *J =* 6.9 Hz, 3H). |
| RZ011 | 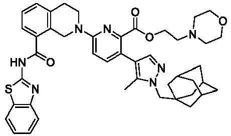 | MS(ESI) m/z = 772.6 [M+H]⁺; |
| | | ¹H NMR (400 MHz, DMSO-*d*₆) δ 8.12 (s, 1H), 7.81 - 7.70 (m, 2H), 7.61-7.55 (m, 2H), 7.44 - 7.40 (m, 1H), 7.3-7.23(m, 1H), 7.19 (td, *J* = 7.4, 1.6 Hz, 1H), 7.09 - 6.95 (m, 2H), 4.43 (t, *J =* 6.0 Hz, 2H), 4.31 (d, *J* = 17.0 Hz, 1H), 4.07 (d, *J* = 17.2 Hz, 1H), 3.96 - 3.88 (m, 2H), 3.67 - 3.51 (m, 7H), 3.15 (t, *J =* 5.9 Hz, 2H), 2.90 - 2.86 (m, 2H), 2.71 (s, 2H), 2.48 -2.23(m, 4H), 1.97-1.93 (m, 3H), 1.61 - 1.47 (m, 12H). |
| RZ013 | 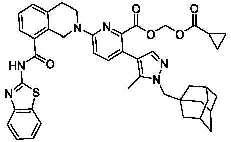 | MS(ESI) m/z = 757.6 [M+H]⁺; |
| RZ014 | 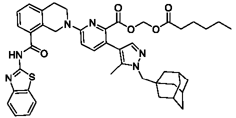 | MS(ESI) m/z = 787.8 [M+H]⁺; |
| RZ015 | 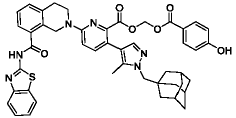 | MS(ESI) m/z = 809.8 [M+H]⁺; |
| RZ016 | 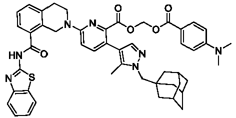 | MS(ESI) m/z = 836.7 [M+H]⁺; |
| RZ017 | 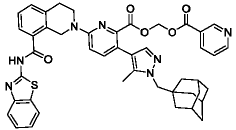 | MS(ESI) m/z = 794.7 [M+H]⁺; |
| RZ018 | 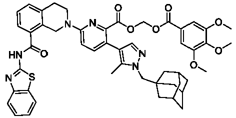 | MS(ESI) m/z = 883.5 [M+H]⁺; |
| RZ019 | 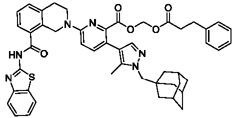 | MS(ESI) m/z = 821.4 [M+H]⁺; |
| RZ020 | 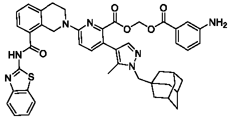 | MS(ESI) m/z = 808.7 [M+H]⁺; |
| RZ021 | 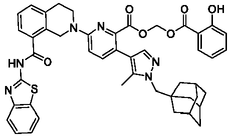 | MS(ESI) m/z = 836.7 [M+H]⁺; |
| RZ022 | 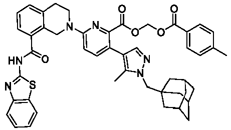 | MS(ESI) m/z = 807.8 [M+H]⁺; |
| RZ023 | 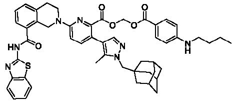 | MS(ESI) m/z = 864.7 [M+H]⁺; |
| RZ024 | 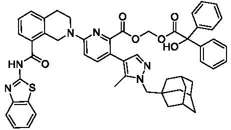 | MS(ESI) m/z = 899.7 [M+H]⁺; |
| RZ025 | 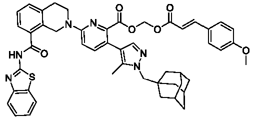 | MS(ESI) m/z = 849.9 [M+H]⁺; |

### Synthesis of Compound RZ002

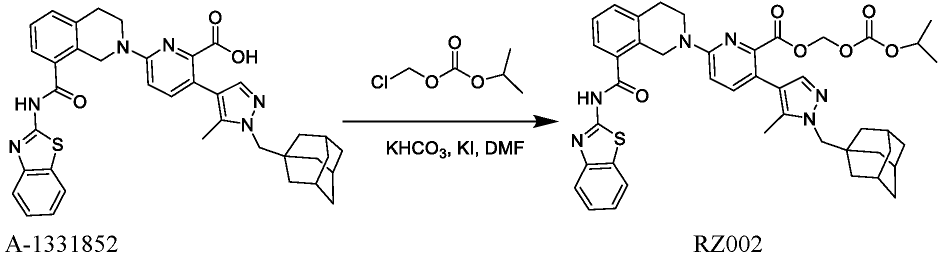

Step: To a solution of compound A-1331852 (30 mg, 0.04554 mmol, 1.00 eq) in DMF (0.8 mL), KHCO₃ (17 mg, 3.73 eq) was added. The mixture was stirred for 30 min at room temperature, followed by the addition of a solution of chloromethyl isopropyl carbonate (8 mg, 1.20 eq) in 0.7 mL DMF. The reaction mixture was heated to 60 °C (external oil bath temperature) and stirred for 4.5 h. The resultant was diluted with ethyl acetate, washed with water, dried and purified by column chromatography.

¹HNMR (400 MHz, Chloroform-d) δ 10.80 (s, 1H), 7.85 (m, 1H), 7.55 (d, 1H), 7.42-7.45 (m, 2H), 7.30-7.35 (m, 4H), 7.21-7.24 (m, 1H), 6.94 (d, 1H), 5.77 (s, 2H), 5.00(s, 2H), 4.86-4.89 (m, 1H), 4.03-4.06 (m, 2H), 3.71 (s, 2H), 3.04 (t, 2H), 2.09 (s, 3H), 1.95-2.05 (m, 3H), 1.59-1.71 (m, 12H), 1.24-1.33 (m, 6H).

### Synthesis of Compound RZ006

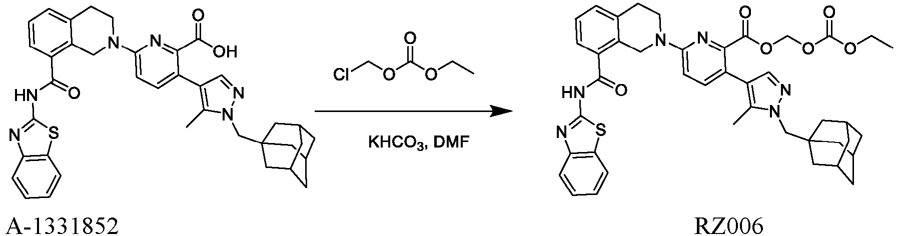

Step: To a solution of compound A-1331852 (50 mg, 0.07589 mmol, 1.00 eq) in DMF (1.0 mL), KHCO₃ (31 mg, 4.08 eq) was added. The mixture was stirred for 20 min at room temperature, followed by the addition of a solution of chloromethyl ethyl carbonate (SM10, 13 mg, 1.24 eq) in 0.8 mL DMF. The reaction mixture was heated to 60 °C (external oil bath temperature) and stirred for 5.0 h. The resultant was diluted with ethyl acetate, washed with water, dried, filtered, concentrated, and purified by column chromatography.

¹HNMR (400 MHz, Chloroform-d) δ 10.80 (s, 1H), 7.85 (m, 1H), 7.55 (d, 1H), 7.41-7.45 (m, 2H), 7.29-7.35 (m, 4H), 7.22-7.24 (m, 1H), 6.94 (d, 1H), 5.77 (s, 2H), 5.00(s, 2H), 4.17-4.23 (q, 2H), 4.02-4.05 (m, 2H), 3.71 (s, 2H), 3.04 (t, 2H), 2.09 (s, 3H), 1.95-2.05 (m, 3H), 1.58-1.71 (m, 12H), 1.24-1.35 (m, 3H).

According to the same method as the above examples, the following example compounds in Table 2 were prepared using commercially available compounds or by referring to the preparation methods of the intermediate compounds as indicated.

**Table 2**

| Compound ID | Structure | H NMR or MS |
|---|---|---|
| RZ010 | 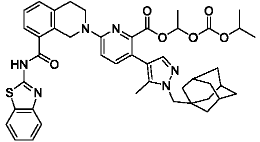 | MS(ESI) m/z = 787.1 [M+H]⁺; |
| | | ¹H NMR (400 MHz, DMSO-*d*₆) δ 8.12 (s, 1H), 7.94 (q, *J* = 5.2 Hz, 1H), 7.81 - 7.70 (m, 2H), 7.61 (td, *J* = 8.0, 1.3 Hz, 2H), 7.44 (dq, *J* = 8.1, 1.1 Hz, 1H), 7.32 (dd, *J* = 7.6, 1.7 Hz, 1H), 7.19 (td, *J =* 7.4, 1.6 Hz, 1H), 7.06 (d, *J* = 7.9 Hz, 1H), 6.99 (td, *J* = 7.5, 1.2 Hz, 1H), 3.62(s, 2H), 4.93 (m, 1H), 4.31 (d, *J =* 17.0 Hz, 1H), 4.07 (d, *J* = 17.2 Hz, 1H), 3.96 - 3.88 (m, 2H), 3.59 (s, 1H), 2.90 (ddd, *J* = 6.8, 4.3, 1.0 Hz, 2H), 2.71 (s, 2H), 1.97 (pt, *J* = 5.9, 4.2 Hz, 3H), 1.77 (d, *J* = 5.2 Hz, 3H), 1.61 - 1.47 (m, 12H), 1.35 (d, *J* = 5.8 Hz, 3H), 1.30 (d, *J =* 6.0 Hz, 3H). |
| RZ026 | 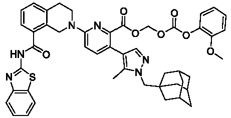 | MS(ESI) m/z = 839.7 [M+H]⁺; |
| RZ027 | 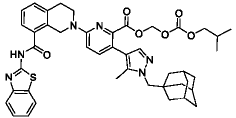 | MS(ESI) m/z = 789.7 [M+H]⁺; |

### Synthesis of Compound RZ201

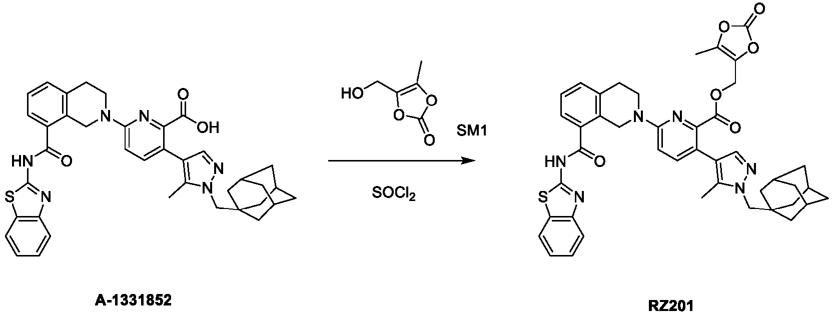

Step: To a solution of compound A-1331852 (50 mg, 0.07589 mmol, 1.00 eq) in DMF (5.0 mL), thionyl chloride (0.1 mL) was added. The mixture was stirred for 30 min at room temperature, followed by the addition of a solution of 4-(hydroxymethyl)-5-methyl-1,3-dioxol-2-one (SM1, 10 mg, 1.0 eq). The reaction mixture was stirred at room temperature for 3 h. The resultant was diluted with dichloromethane, washed with water, dried, concentrated, and purified by column chromatography.

MS(ESI) m/z = 771.3 [M+H]⁺;

¹H NMR (400 MHz, DMSO-*d*₆) δ 8.12 (s, 1H), 7.81 - 7.70 (m, 2H), 7.61 (td, *J* = 8.0, 1.2 Hz, 2H), 7.44 (dq, *J =* 8.1, 1.1 Hz, 1H), 7.32 (dd, *J* = 7.7, 1.7 Hz, 1H), 7.19 (td, *J* = 7.4, 1.6 Hz, 1H), 7.06 (d, *J* = 7.9 Hz, 1H), 6.99 (td, *J* = 7.5, 1.2 Hz, 1H), 5.05 (q, *J* = 1.1 Hz, 2H), 4.31 (d, *J* = 17.0 Hz, 1H), 4.07 (d, *J* = 17.2 Hz, 1H), 3.96 - 3.88 (m, 2H), 3.59 (s, 2H), 2.90 (ddd, *J* = 6.8, 4.3, 1.0 Hz, 2H), 2.71 (s, 2H), 2.08 (t, *J =* 1.0 Hz, 3H), 1.97 (pt, *J =* 5.9, 4.2 Hz, 3H), 1.61 - 1.47 (m, 13H).

According to the same method as the above examples, the following example compounds in Table 3 were prepared using commercially available compounds or by referring to the preparation methods of the intermediate compounds as indicated.

**Table 3**

| Compound ID | Structure | H NMR or MS |
|---|---|---|
| RZ202 | 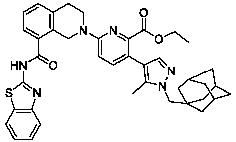 | MS(ESI) m/z = 687.5 [M+H]⁺; |
| | | ¹H NMR (400 MHz, DMSO-*d*₆) δ 8.12 (s, 1H), 7.76 - 7.71 (m, 2H), 7.62 - 7.58 (m, 2H), 7.44 (dd, *J =* 8.0, 1.0 Hz, 1H), 7.32 (dd, *J =* 7.7, 1.7 Hz, 1H), 7.18 (dd, *J* = 7.4, 1.6 Hz, 1H), 7.07 (s, 1H), 7.00 (dd, *J =* 7.4, 1.2 Hz, 1H), 4.36 - 4.23 (m, 3H), 4.07 (d, *J* = 17.2 Hz, 1H), 3.96 - 3.91 (m, 2H), 3.59 (s, 2H), 2.91 - 2.86 (m, 2H), 2.71 (s, 3H), 2.05 - 1.96 (m, 3H), 1.55 - 1.47 (m, 12H), 1.29 (d, *J* = 6.4 Hz, 3H). |
| RZ203 | 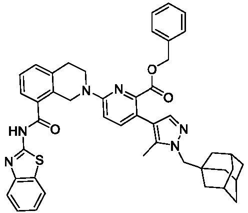 | MS(ESI) m/z = 687.5 [M+H]⁺; |
| RZ204 | 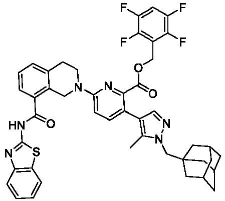 | MS(ESI) m/z = 687.5 [M+H]⁺; |
| RZ205 | 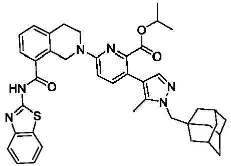 | MS(ESI) m/z = 687.5 [M+H]⁺; |
| RZ206 | 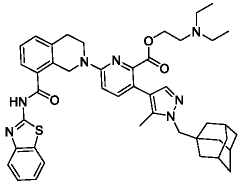 | MS(ESI) m/z = 758.5 [M+H]⁺; |

### Synthesis of Compound RZ301

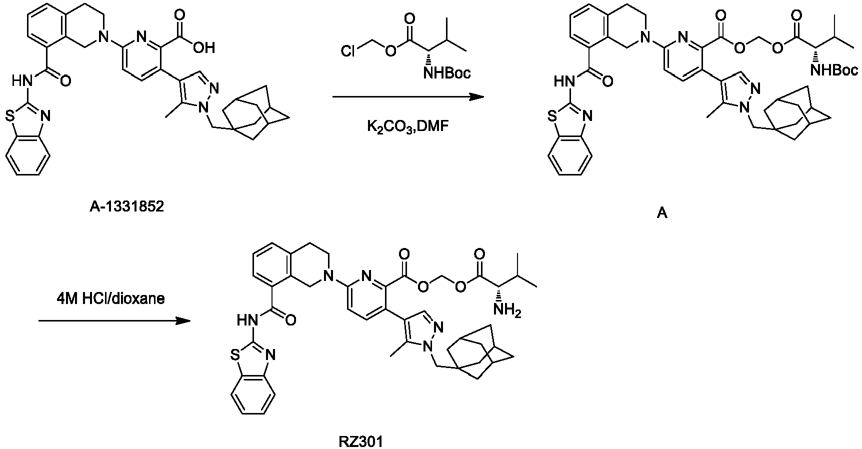

Step 1: To a solution of compound A-1331852 (50 mg, 0.076 mmol, 1.00 eq) in DMF (1.0 mL), potassium carbonate (16 mg) was added. The mixture was stirred for 30 min at room temperature, followed by the addition of chloromethyl N-{[(2-methylprop-2-yl)oxy]carbonyl}-L-valine ester (24 mg, 1.2 eq) was added. The reaction mixture was heated to 50°C (external oil bath temperature) and stirred for 4 h. After cooling, the reaction was quenched with water. The resultant was extracted with ethyl acetate, washed with saturated NaCl solution, dried, concentrated, and subjected to column chromatography to obtain compound A (50 mg, yield: 74.1%).

Step 2: To a solution of compound A (50 mg, 0.056 mmol) in dioxane (2 mL), 4M hydrochloric acid/dioxane solution (0.1 mL) was added. The mixture was stirred at room temperature for 1 h. The resultant was diluted with dichloromethane and the pH was adjusted to 7 with saturated sodium bicarbonate solution, extracted, dried, concentrated and purified by column chromatography.

MS(ESI) m/z = 788.5 [M+H]⁺;

¹H NMR (400 MHz, DMSO-*d*₆) δ 8.90 (d, *J =* 7.4 Hz, 2H), 7.90 (s, 1H), 7.77 (t, *J =* 8.2 Hz, 1H), 7.73 (d, *J* = 7.9 Hz, 1H), 7.61 (td, *J =* 8.0, 1.3 Hz, 2H), 7.44 (dq, *J =* 8.1, 1.1 Hz, 1H), 7.32 (dd, *J =* 7.7, 1.7 Hz, 1H), 7.19 (td, *J =* 7.4, 1.6 Hz, 1H), 7.09 - 7.03 (m, 2H), 7.03 (d, *J* = 3.3 Hz, 1H), 6.99 (td, *J* = 7.5, 1.2 Hz, 1H), 4.31 (d, *J* = 17.0 Hz, 1H), 4.07 (d, *J =* 17.2 Hz, 1H), 3.96 - 3.88 (m, 2H), 3.55 (td, *J =* 7.2, 6.4 Hz, 1H), 2.90 (ddd, *J =* 6.8, 4.3, 1.0 Hz, 2H), 2.71 (s, 2H), 2.27 (dt, *J =* 13.0, 6.5 Hz, 1H), 1.97 (pt, *J* = 5.9, 4.2 Hz, 3H), 1.61 - 1.47 (m, 15H), 1.08 (d, *J =* 6.5 Hz, 3H), 1.02 (d, *J =* 6.5 Hz, 3H).

### Example 2. Higher Level of Esterase in Senescent Cells Compared to Normal Cells

In this example, human non-small cell lung cancer cell line (A549 cells), human retinal pigment epithelial cells (RPE cells), and human pulmonary fibrosis cell line (IMR90 cells) were selected. This study aimed to determine whether the intracellular esterase level in senescent cells is higher than that in normal cells, thereby evaluating the potential advantage of prodrug release in senescent cellular environment.

Cells were planted in 100 mm culture dishes and induced to senesce using bleomycin (10 µM) and etoposide (10 µM). Both normal and senescent cells were counted after digestion. The cells were lysed by ultrasonication and then centrifuged for 10 min. The supernatant was collected and kept on ice. Esterase activity was measured according to the kit instructions. The formula for calculating CarE enzyme activity is as follows: CarE enzyme activity (U/104 cells) = (ΔA sample tube - ΔA blank tube) × Vtotal reaction÷0.5÷ (200÷Vtotal cell sample × Vsample)÷T×F

Vₛₐₘₚₗₑ: Volume of the sample added, 0.01 mL; T: Reaction time, 5 min; 200: Total number of cells, 2 million; V_{total cell sample}: Volume of extraction liquid added to the cells, 0.2 mL; V_{total reaction}: Total volume of the reaction system, 0.2 mL; F: Dilution factor.

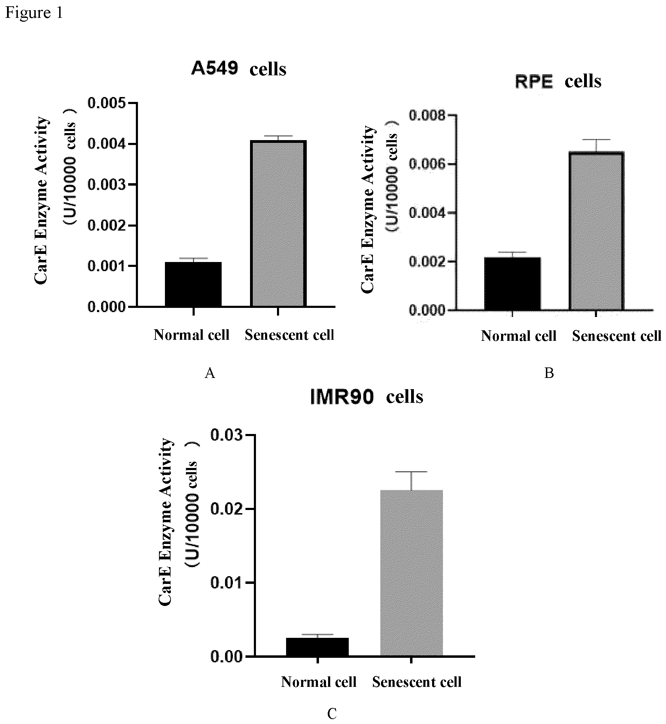

As shown in Figures 1A, 1B, and 1C, the level of intracellular esterase in senescent cells was significantly higher than that in normal cells.

### Example 3. More Specific Killing Effect of The Prodrug on Senescent Cells Than A-1331852

In this example, human non-small cell lung cancer cell line (A549 cells), human embryonic lung fibroblast cell line (IMR90 cells), human retinal pigment epithelial cells (RPE cells), mouse chondrocytes, and mouse skin fibroblasts were selected. The cells above were induced to obtain senescent cells by etoposide (10µM). The effect of the target compounds RZ001-RZ006 and RZ201 on the viability of normal and senescent cells above was detected, thereby evaluating the specific killing effect of the prodrugs RZ001-RZ006 and RZ201 on senescent cells above.

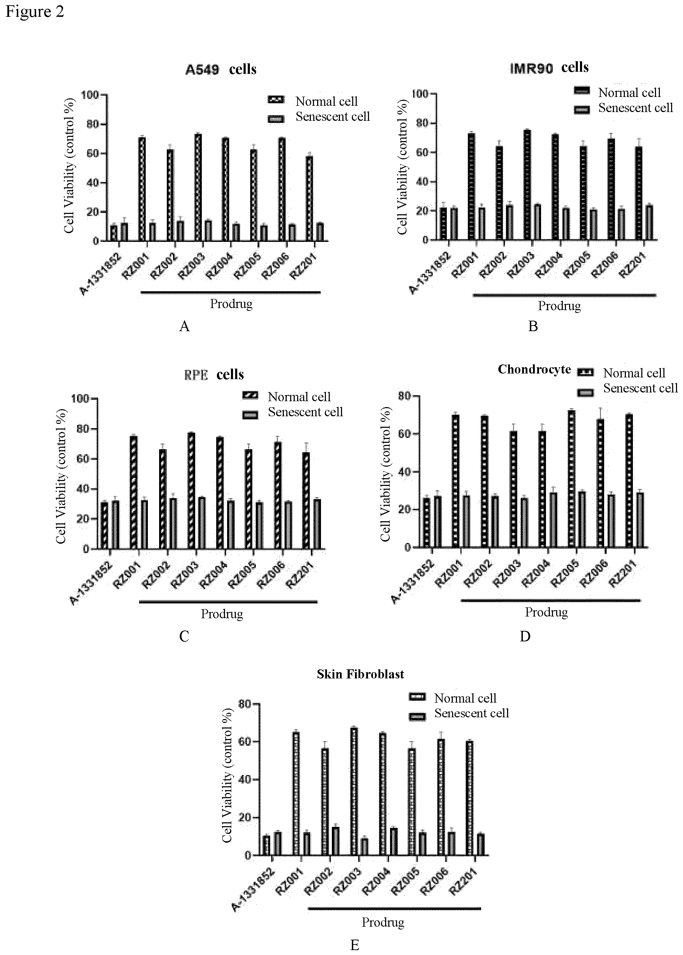

Briefly, senescent and normal cells above were selected and plated separately (96-well plates). After adding the compounds RZ001-RZ006 and RZ201 (concentration: 1µM) for 72 hours, CellTiter-Glo^{®} Luminescent Cell Viability Assay Kit (Promega) was used to detect cell viability, and the results were shown in Figures 2A to 2E.

As shown in Figures 2A to 2E, in addition to killing senescent cells, A-1331852 exhibits significant cytotoxicity against normal cells. In contrast, the compounds RZ001-RZ006 and RZ201 demonstrate cytotoxicity primarily against senescent cells without significant killing effects on normal cells. This indicates that RZ001-RZ006 and RZ201 possess superior specificity and safety.

Furthermore, the senescent cell clearance index (senolytic index) was also calculated for both normal and senescent A549 cells, as shown in Table 4. A higher index indicates greater specificity in eliminating senescent cells and better safety.

**Table 4. Senolytic Index**

| Number | Molecular Weight | senescent cell clearance index (Senolytic Index) |
|---|---|---|
| 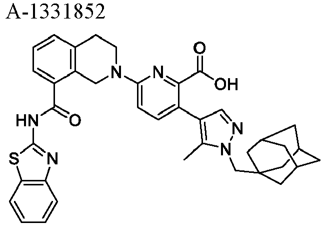 | 658.81 | D |
| 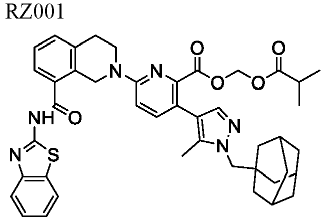 | 758.94 | A |
| 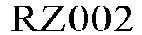 | 774.94 | B |
| 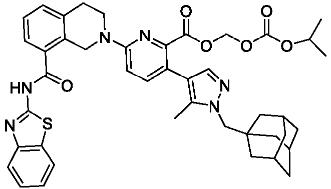 | | |
| 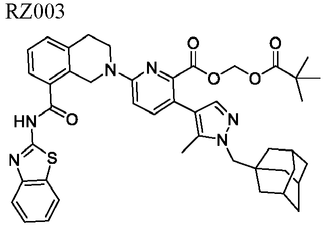 | 772.97 | B |
| 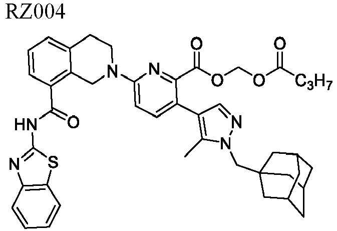 | 758.94 | B |
| 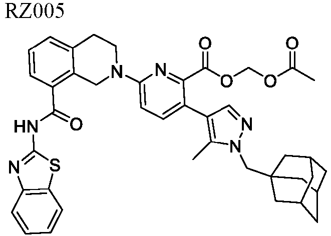 | 730.88 | B |
| 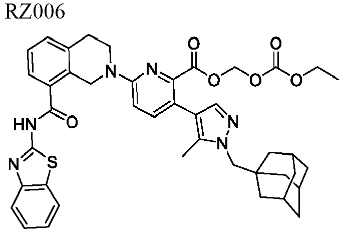 | 760.91 | B |
| 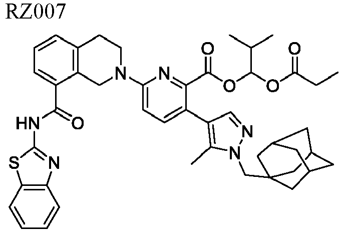 | 786.99 | B |
| 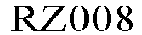 | 714.93 | F |
| 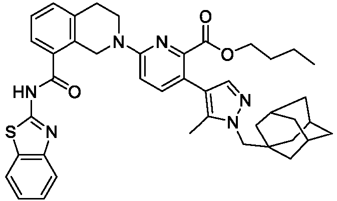 | | |
| 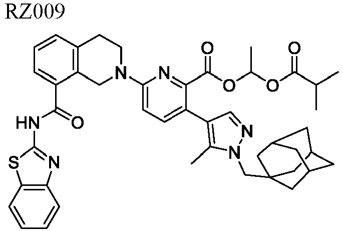 | 772.96 | B |
| 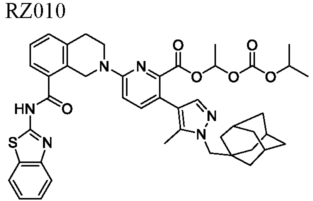 | 788.96 | C |
| 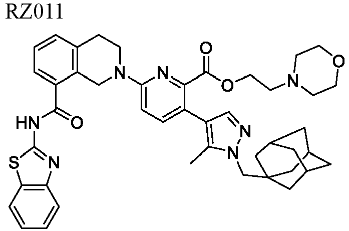 | 771.98 | F |
| 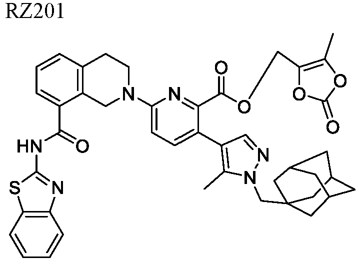 | 770.90 | A |
| 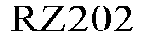 | 686.88 | F |
| 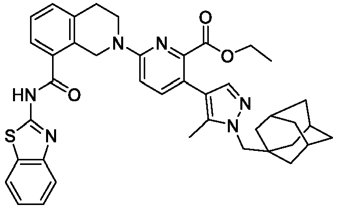 | | |
| 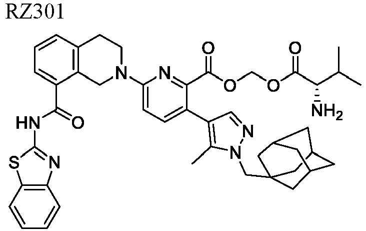 | 787.98 | B |

| | | |
|---|---|---|
| Note: A represents more than 200; B represents 100 to 200; C represents 50 to 100; D represents 30 to 50; E represents 10 to 30; F represents less than 10. | | |

### Example 4: Efficacy in Animal Model of Hyperoxia-Induced Retinopathy

The efficacy of the prodrugs RZ001-RZ006 as well as RZ201 were investigated in a mouse model of oxygen-induced retinopathy (OIR) which to some extent indicates an *in vivo* model of retinopathy of prematurity (ROP), diabetic retinopathy, and wet age-related macular degeneration.

From postnatal Day 7 (P7) to Day 12 (P12), C57Bl/6 mouse pups and their CD1 foster mothers were exposed to hyperoxia (75% O₂). At P12, the animals were injected intravitreally with 1µl of the test composition formulated in 1% DMSO, 10% Tween 80, and 20% PEG-400 (200 µM the compound) and returned to room air until Day 17 (P17). On P17, the eyeballs were enucleated and the retinas were dissected for vascular staining. To determine the area of avascularity or angiogenesis, the retinas were flattened and stained with isolectin B4 (IB4).

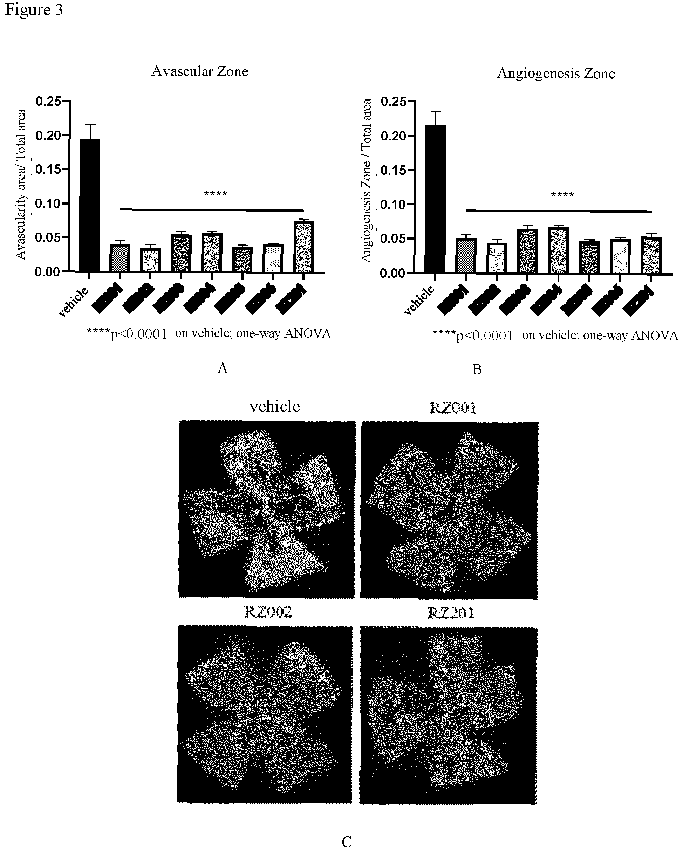

Figure 3 shows that intravitreal (IVT) administration of RZ001-RZ006 and RZ201 resulted in statistically significant improvements in avascular zone and angiogenesis.

### Example 5: Efficacy in Animal Model of Diabetes-Induced Retinopathy

The efficacy of the compounds of the present disclosure was studied in a mouse model of diabetic retinopathy by multiple administrations of streptozotocin (STZ). C57BL/6J mice at 6 to 7 weeks were weighed and their baseline blood glucose was measured (Sino). STZ (Sigma-Alderich) was injected intraperitoneally into the mice at 55 mg/Kg for five consecutive days. Age-matched controls were injected with buffer only. Blood glucose was measured again one week after the last STZ injection. 1µL of the test compound (200 µM, a suspension formulated in 0.015% polysorbate 80, 0.2% sodium phosphate, 0.75% sodium chloride, pH 7.2) was injected intravitreally into the STZ-treated diabetic C57BL/6J mice at 8 and 9 weeks after STZ administration. 10 weeks after STZ treatment, the retinal Evans blue penetration assay was performed.

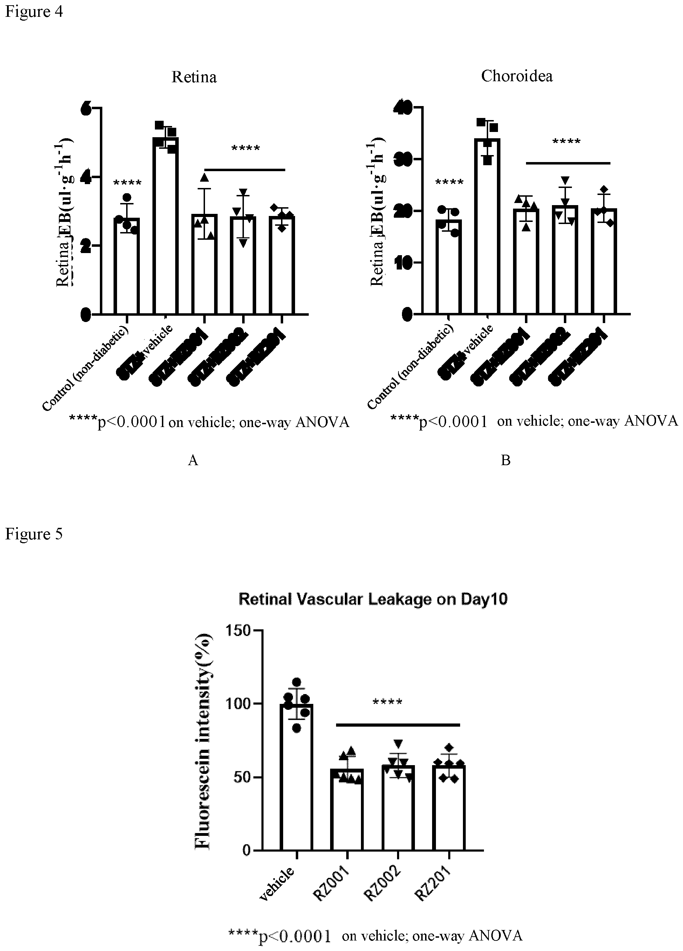

Figure 4 shows intravitreal (IVT) administration of RZ001, RZ002 and RZ201 resulted in improved vascular permeability in both the retinal and choroidal vascular leakage at this dose level.

### Example 6: Efficacy in CNV Animal Model of Wet-Age-Related Macular Degeneration

In this example, the efficacy of the compounds of the present disclosure was tested on wet-age-related macular degeneration in a CNV animal model.

Male mice were administered 0.5% probacaine hydrochloride (Alcon) for local anesthesia, and 1% tropicamide to dilate the pupils of the mice. Laser cauterization was performed using a laser photocoagulator (Novus Varia, LUMENIS) with a slit lamp delivery system. And artificially torn adherent glass coverslips were used as contact lenses. Bleeding spots caused by laser were not included in the statistical data.

Figure 5 shows that intravitreal (IVT) administration of RZ001, RZ002 and RZ201 resulted in statistically significant improvements in fluorescence intensity indicators.

### Example 7: Efficacy in Animal Model of Idiopathic Pulmonary Fibrosis

In this example, the efficacy of the compounds of the present disclosure was tested on idiopathic pulmonary fibrosis in an idiopathic pulmonary fibrosis animal model.

Briefly, anesthetized 6-week-old SD rats were fixed on a foam board with medical tape. Firstly, the skin on the surface of the trachea was cut with surgical scissors to fully expose the trachea, and a 100-microlitre micro-syringe containing a certain volume of bleomycin (BLM 5 mg/kg) solution drawn based on the body weight of the rat was inserted into the trachea through the oral cavity to administer by slow bolus injection. After administration of the BLM solution, the foam plate was immediately rotated and shaken to distribute the BLM solution as evenly as possible in the lung tissue. Then the skin on the surface of the trachea was sutured with surgical sutures and about 0.5 to 1.0 mL of penicillin solution was dripped onto the epidermis to prevent infection of the surgical wound. After the 7th day of modelling, the compound solution of the present disclosure was administered via intraperitoneal (IP) injection (5 mg/kg) according to the weight of the rat for 3 consecutive weeks.

Rats were euthanized by carbon dioxide at Day 28, the lung tissues were removed, the left alveolar lavage fluid was collected for white blood cell (WBC) counting, the right lung tissue portion was used for hydroxyproline (HYP) assay, and the remaining lung tissues were stained for pathology by H&E staining and Masson staining.

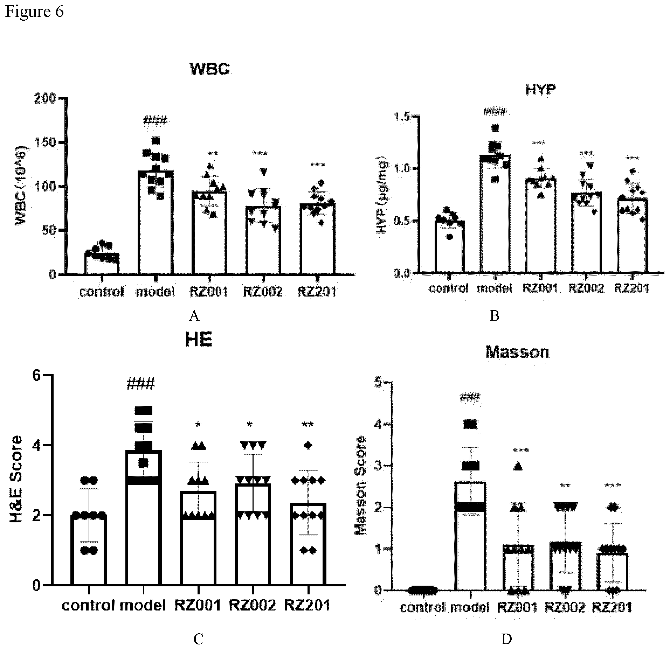

Figure 6 shows that administration of RZ001, RZ002 and RZ201 via intraperitoneal (IP) injection resulted in statistically significant improvements in WBC, HYP and pathology scores in the IPF model.

### Example 8: Efficacy in Animal Models of Osteoarthritis

In this example, the efficacy of the compounds of the present disclosure was tested on osteoarthritis in an osteoarthritis animal model.

The osteoarthritis animal model was constructed by the following steps. The mice were anesthetized with isoflurane, and fixed. The hair on the right hind limb of the mice was shaved and the skin was disinfected with alcohol, and the skin of the joints was cut. Under an optical microscope, the inside of the right knee joint was incised longitudinally with a blade, without cutting the patellar ligament. The patellar ligament was pulled to one side with constant hemostasis by compression with sterile cotton swabs such that the joint cavity was fully exposed . Saline was added dropwise in time to avoid the drying up of the joint cavity. The excess muscle tissue was gently separated with a blunt forceps to expose the meniscus ligament. The anterior cruciate ligament was found in the depth of the joint cavity and cut with micro scissor. Then the wound was disinfected with penicillin solution, the muscle was sutured with sterile needle and thread, and the skin tissue was finally sutured. The efficacy of the drug was determined by intra-articular injection administration. At the endpoint of the test, continued pain threshold of the plantar was detected, and the bone joints were stained with safranin fast green.

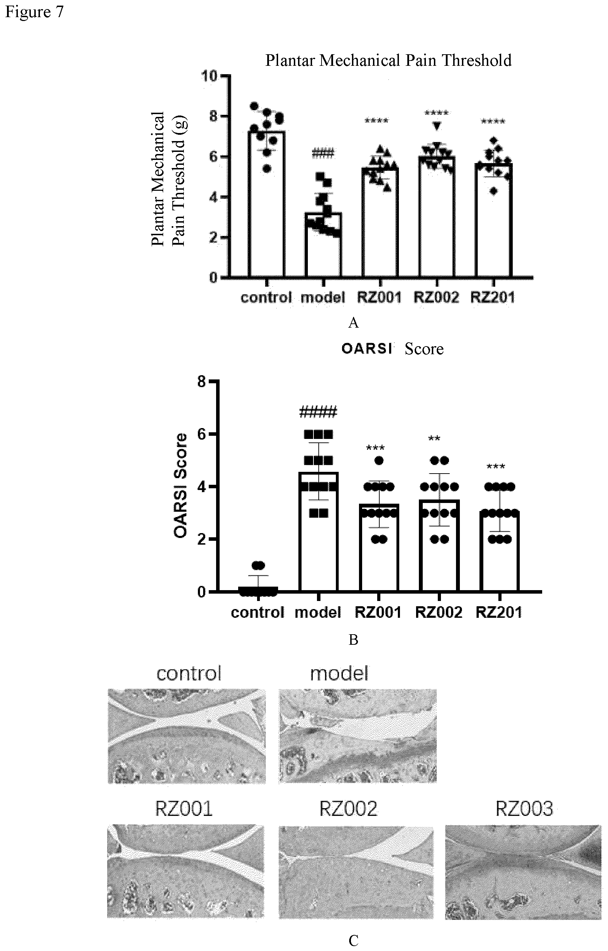

Figure 7 shows that administration of RZ001, RZ002 and RZ201 via intra-articular injection resulted in statistically significant improvements in plantar mechanical pain threshold and pathology scores of safranin fast green staining in an osteoarthritis (OA) model.

### Example 9: Efficacy in Animal Model of Alzheimer's Disease

In this example, the efficacy of the compounds of the present disclosure was tested on Alzheimer's disease in an animal model of Alzheimer's disease.

Briefly, a transgenic mouse model of APP×PS1 (purchased from Cyagen Biosciences) was selected and the compound of the present disclosure was administered by intraperitoneal injection for 11 consecutive weeks, twice a week. Alterations in amyloid in the cerebral cortex of the mice were observed.

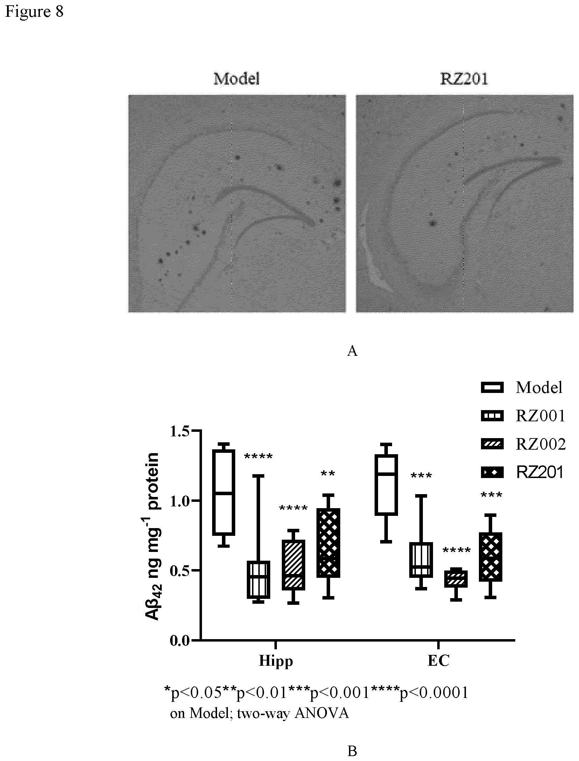

Figure 8 shows that administration of RZ001, RZ002 and RZ201 via intraperitoneal injection resulted in statistically significant improvements in Aβ42 pathogenic protein indicators in an AD mouse model.

### Example 10: Efficacy in Animal Models of Hepatic Fibrosis

In this example, the efficacy of the compounds of the present disclosure was tested on hepatic fibrosis in an animal model of hepatic fibrosis. Briefly, C57BL/6N mice, male, 7 to 8 weeks old, 24 to 28 g of body weight, were used. The mouse model of hepatic fibrosis was induced by intraperitoneal injection of CCl₄ solution at a dosage of 1 mL/kg, with a dosing frequency of 2 times/week, and a modelling cycle of 6 weeks. Dosing via intraperitoneal injection was started from the third week of modelling twice a week. At the end of the test, serum was collected, and liver tissue was taken for Masson staining index assay.

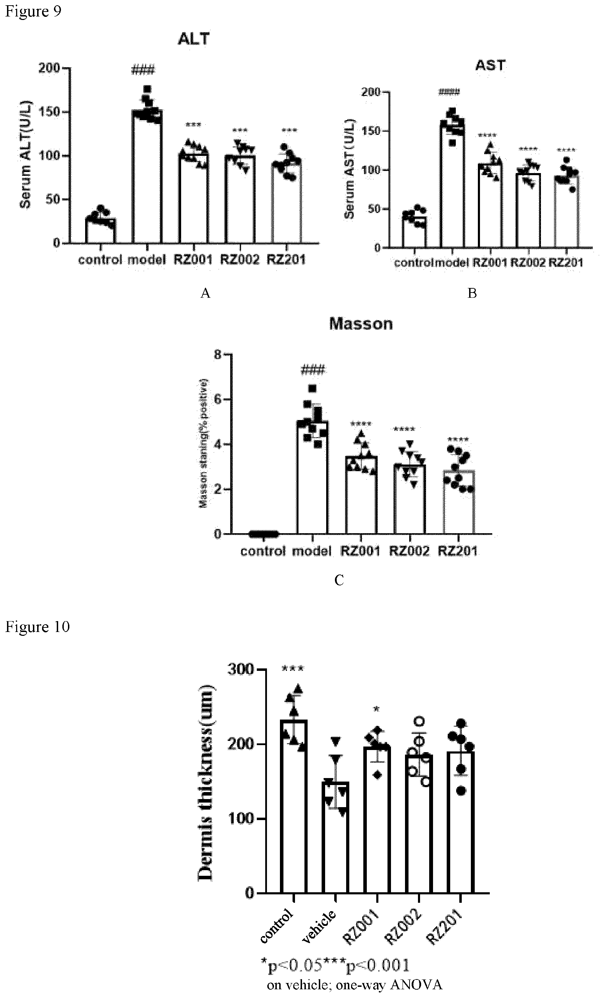

Figure 9 shows that administration of RZ001, RZ002 and RZ201 via intraperitoneal (IP) injection resulted in statistically significant improvements in serum aspartate aminotransferase (AST) and alanine aminotransferase (ALT) levels and pathology scores in a liver fibrosis model.

### Example 11: Efficacy on Aging Skin

In this example, aged female C57bl/6J mice (22 months old) were used. The drug was administered by skin application at twice a week for 6 consecutive weeks. Improvement in the epidermal thickness of the skin was observed in aged mice.

Figure 10 shows that administration of RZ001, RZ002 and RZ201 by applying resulted in statistically significant improvements in epidermal thickness scores of the skins in a naturally aging mouse model.

### Example 12: Efficacy in Renal Fibrosis Animal Model

In this example, the efficacy of the compound of the present disclosure was tested on renal fibrosis in an animal model of renal fibrosis. Briefly, C57BL/6J mice, male, 7 to 8 weeks old, were used. The mice were anaesthetized, made to lie supine on a foam board, and disinfected with iodophor, and a longitudinal incision was made in the skin in the middle of the lower abdomen to locate the white line of the abdominal muscle, where the abdominal muscle, fascia and peritoneal layer were dissected. After exposing the surgical field, the kidneys were exposed by pushing the stomach and mesentery of the mice to the right side with a gauze soaked with saline. The model of renal fibrosis was constructed by locating the transparent ureter from the renal pelvis along the renal artery, separating the left ureter, ligating the upper 1/3 of the left ureter, suturing the incision layer by layer, and finally disinfecting with iodophor. Dosing via intraperitoneal injection was started from the 2nd week of modelling. At the end of the experiment, the mouse serum was collected and kidney tissues were taken for Masson staining index assay.

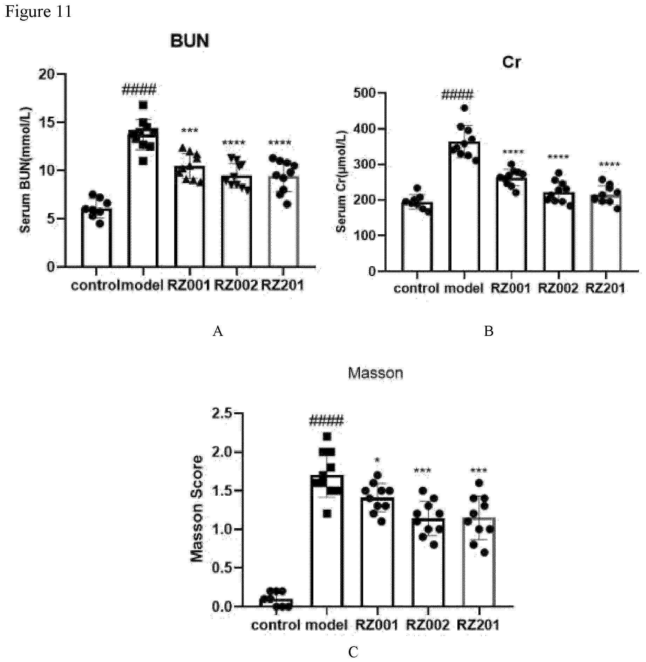

Figure 11 shows that administration of RZ001, RZ002 and RZ201 via intraperitoneal (IP) injection resulted in statistically significant improvements in serum creatinine (Cr) and urea nitrogen (BUN) levels and pathology in a renal fibrosis model.

### Example 13: Efficacy in Rabbit Ear Hypertrophic Scar Model

In this example, the efficacy of the compounds of the present disclosure was tested on hypertrophic scars in a rabbit ear hypertrophic scar animal model. Briefly, 10 New Zealand white rabbits, male, 3 months old, were used. The New Zealand rabbits were anaesthetized with sodium pentobarbital and six circular full-thickness wounds with a diameter of 10 mm were made on the ventral surface of each ear by removing the epidermis, dermis and perichondrium to expose the cartilage. The rabbits were randomly divided into control and RZ001 experimental groups. On postoperative Day 14, after complete re-epithelialization of the wound surface, the compound (150 µM, 100 µL, dissolved in DMSO solution) or negative control DMSO 100 µL was injected into the center of each lesion from the wound edge once a week for a total of 4 injections. Improvement of scar formation by the compound was assessed by SEI (Scar Excess Index).

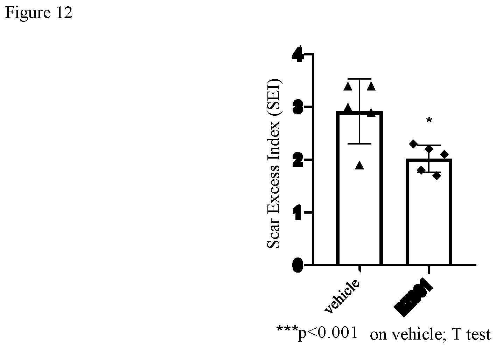

Figure 12 shows that administration of RZ001 resulted in a statistically significant improvement in scar excess index in rabbit ear hypertrophic scar model.

## Claims

1. A compound having the structure represented by formula I, or a pharmaceutically acceptable salt, solvate, hydrate, polymorph, co-crystal, tautomer, stereoisomer, or isotope labeled compound thereof, 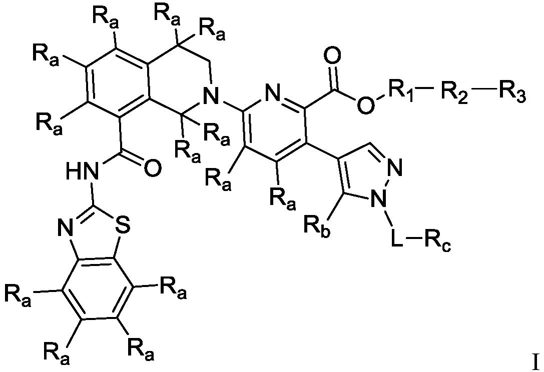 wherein,
R₁ is selected from substituted or unsubstituted C₁-C₁₀ alkylene;
R₂ is selected from the group consisting of -O-C(O)- and -O-C(O)-O-;
R₃ is selected from the group consisting of substituted or unsubstituted C1-C10 chain alkyl, substituted or unsubstituted C3-C20 cycloalkyl, substituted or unsubstituted C2-C20 chain alkenyl, substituted or unsubstituted C2-C20 chain alkynyl, substituted or unsubstituted C6-C20 aryl, substituted or unsubstituted C1-C20 heteroaryl, and substituted or unsubstituted C2-C20 heteroalicyclic group;
or R₁ is absent or is selected from substituted or unsubstituted C₁-C₁₀ alkylene, R₂ is absent, and R₃ is selected from substituted or unsubstituted C2-C20 heteroalicyclic group;
R_{c} is selected from substituted or unsubstituted adamantyl;
alternatively, the substituent in the substitution is selected from the group consisting of halogen atom, cyano, nitro, C6-C20 aryl, C1-C20 heteroaryl, C1-C10 chain alkyl, C1-C10 chain alkoxy, C6-C20 aryloxy, C1-C20 heteroalicyclic group preferably C1-C10 heteroalicyclic group, amino, hydroxyl, mercapto, phosphate group, -OC(O)R₆, -ONR₆R₇, or -NR₆R₇, R₆ and R₇ are independently selected from the group consisting of hydrogen, C6-C20 aryl, C1-C20 heteroaryl, C1-C8 chain alkyl, C3-C8 cycloalkyl, C2-C8 chain alkenyl, and C2-C8 chain alkynyl, the aryl and heteroaryl are optionally substituted with halogen atom, hydroxyl, mercapto, amino, nitro, cyano, carboxyl, acyl, C1-C6 alkoxy, C6-C20 aryl, C1-C20 heteroaryl, C2-C20 heteroalicyclic group, C1-C10 alkyl, C3-C8 cycloalkyl, C2-C8 chain alkenyl, or C2-C8 chain alkynyl, wherein optionally the substituents of at least two positions together form an aliphatic ring such as C3-C20 aliphatic ring, a heteroaliphatic ring such as C2-C20 heteroaliphatic ring, an aromatic ring such as C6-C20 aromatic ring, or a heteroaromatic ring such as C1-C20 heteroaromatic ring.

2. The compound according to claim 1, **characterized in that** the compound is represented by formula III, IV or V: 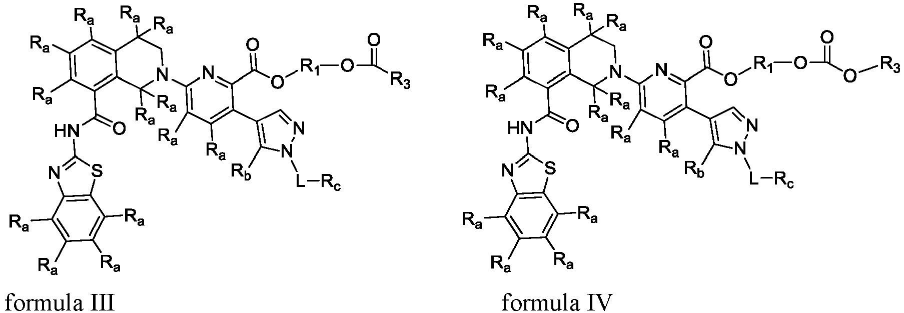 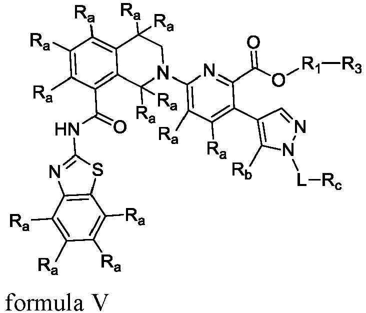 wherein,
in formula III and formula IV, R₁ is independently selected from substituted or unsubstituted C₁-C₁₀ alkylene, preferably, R₁ is independently selected from substituted or unsubstituted C₁-C₆ alkylene, preferably, R₁ is independently selected from substituted or unsubstituted C₁-C₄ alkylene; and Rₐ, R_{b}, R₃, R_{c}, and L are defined as in formula I; and
in formula V, R₁ is absent or independently selected from substituted or unsubstituted C₁-C₁₀ alkylene, preferably, R₁ is independently selected from substituted or unsubstituted C₁-C₆ alkylene, preferably, R₁ is independently selected from substituted or unsubstituted C₁-C₄ alkylene, R₃ is selected from substituted or unsubstituted C2-C20 heteroalicyclic group, and Rₐ, R_{b}, R_{c}, and L are defined as in formula I.

3. The compound according to claim 1 or 2, **characterized in that**,
the C2-C20 heteroalicyclic group is optionally substituted with a substituent selected from the group consisting of halogen atom, hydroxyl, mercapto, amino, nitro, cyano, C1-C10 alkoxy, C1-C10 alkyl, C3-C8 cycloalkyl, C2-C8 chain alkenyl, C2-C8 alkynyl, 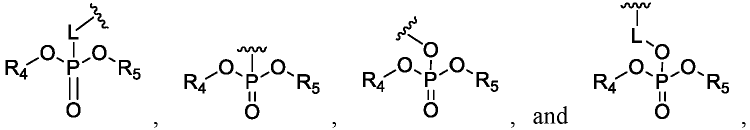 and each R₅ is independently selected from the group consisting of hydrogen and C1-C6 alkyl.

4. The compound according to claim 1 or 2, **characterized in that** the C2-C20 heteroalicyclic group is a C4-C8 heteroalicyclic group;
the C4-C8 heteroalicyclic group is optionally substituted with halogen, -NH₂, -OH, -NO₂, carbonyl, -CH₂OH, carboxyl, methyl, ethyl, propyl, isopropyl, methoxy, ethoxy, propoxy, or isopropoxy.

5. The compound according to any one of claims 1 to 4, **characterized in that** the C2-C20 heteroalicyclic group is selected from a group represented by the following groups: 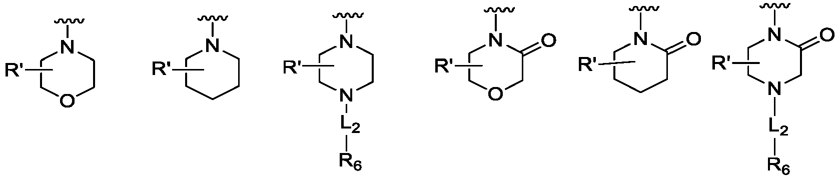 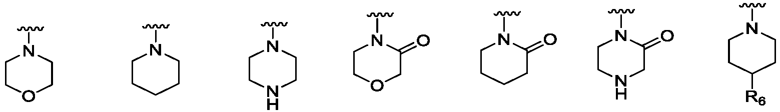 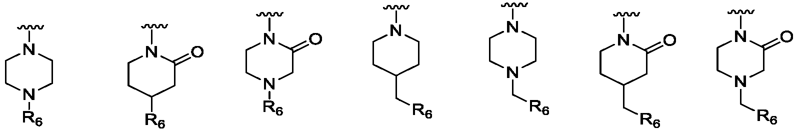 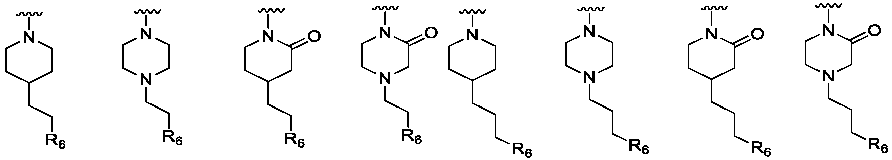 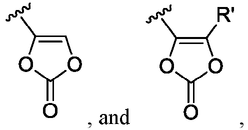
R' each independently represents no substituent, single substituent, or multiple substituents, and each substituent is independently selected from the group consisting of deuterium, hydroxyl, halogen, NH₂, carboxyl (-COOH), 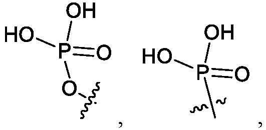
C1-C6 chain alkyl, halogen-substituted C1-C6 chain alkyl, hydroxyl-substituted C1-C6 chain alkyl, amino-substituted C1-C6 chain alkyl, morpholine-substituted C1-C6 chain alkyl, -COO-C1-C6 chain alkyl, cyano, C1-C6 chain alkoxy, C3-C6 cycloalkyl, halogen-substituted C3-C6 cycloalkyl, hydroxyl-substituted C3-C6 cycloalkyl, phenyl, and benzyl;
L₂ is absent, or C1-C6 alkylene, halogen, hydroxyl, or C1-C6 alkoxy-substituted C1-C6 alkylene, preferably methylene, ethylene, or propylene;
R₆ is H, deuterium, halogen, hydroxyl, NH₂, carboxyl (-COOH), -CONH₂, sulfonic acid group (-SO₃H), sulfonyl-SO₂-C1-C6 chain alkyl, 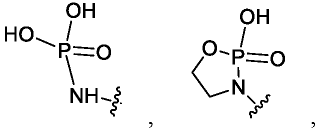 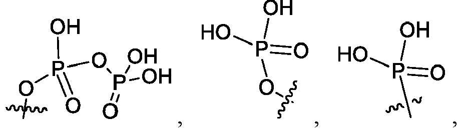 C1-C6 chain alkyl, halogen-substituted C1-C6 chain alkyl, hydroxyl-substituted C1-C6 chain alkyl, amino-substituted C1-C6 chain alkyl, C3-C6 cycloalkyl, halogen-substituted C3-C6 cycloalkyl, hydroxyl-substituted C3-C6 cycloalkyl, phenyl, or benzyl.

6. The compound according to any one of claims 1 to 5, **characterized in that** each Rₐ is hydrogen; and/or
R_{b} is selected from substituted or unsubstituted C1-C6 alkyl, preferably substituted or unsubstituted C1-C3 alkyl, and more preferably methyl; and/or
L is selected from substituted or unsubstituted C1-C6 alkylene, preferably substituted or unsubstituted C1-C3 alkylene, and more preferably methylene.

7. The compound according to any one of claims 1 to 6, **characterized in that** the compound is represented by formula II: 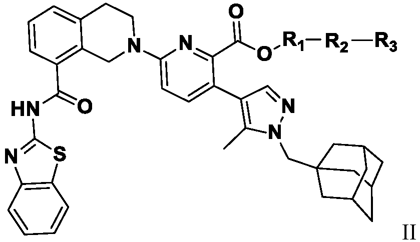 wherein, in formula II, R₁, R₂, and R₃ are defined as in formula I.

8. The compound according to any one of claims 1 to 7, **characterized in that** R₁ is substituted or unsubstituted C1-C6 alkylene, preferably substituted or unsubstituted C1-C3 alkylene, more preferably methylene, and/or
R₃ is selected from the group consisting of substituted or unsubstituted C1-C8 chain alkyl, substituted or unsubstituted C3-C10 cycloalkyl, substituted or unsubstituted C2-C8 chain alkenyl, substituted or unsubstituted C2-C8 chain alkynyl, substituted or unsubstituted C6-C12 aryl, substituted or unsubstituted C2-C12 heteroaryl, or substituted or unsubstituted C1-C12 heteroalicyclic group;
preferably, R₃ is selected from the group consisting of substituted or unsubstituted C1-C6 alkyl, substituted or unsubstituted C6-C12 aryl, substituted or unsubstituted C1-C10 heteroaryl, substituted or unsubstituted C3-C8 cycloalkyl, or substituted or unsubstituted C1-C10 heteroalicyclic group.

9. The compound according to any one of claims 1 to 8, **characterized in that** the substitution is substituted by a substituent selected from the group consisting of halogen atom, cyano, nitro, C6-C20 aryl, C1-C20 heteroaryl, C1-C10 chain alkyl, C1-C10 chain alkoxy, C6-C20 aryloxy, C1-C20 heteroalicyclic group preferably C1-C10 heteroalicyclic group, amino, hydroxyl, mercapto, phosphate group, -OC(O)R₆, -ONR₆R₇, or -NR₆R₇, and R₆ and R₇ are independently selected from the group consisting of hydrogen, C6-C20 aryl, C1-C20 heteroaryl, C1-C8 chain alkyl, C3-C8 cycloalkyl, C2-C8 chain alkenyl, and C2-C8 chain alkynyl, the aryl and heteroaryl are optionally substituted with halogen atom, hydroxyl, mercapto, amino, nitro, cyano, carboxyl, acyl, C1-C6 alkoxy, C6-C20 aryl, C1-C20 heteroaryl, C2-C20 heteroalicyclic group, C1-C10 alkyl, C3-C8 cycloalkyl, C2-C8 chain alkenyl, or C2-C8 chain alkynyl, wherein optionally the substituents of at least two positions together form an aliphatic ring such as C3-C20 aliphatic ring, a heteroalicyclic ring such as C2-C20 heteroaliphatic ring, an aromatic ring such as C6-C20 aromatic ring ,or a heteroaromatic ring such as C1-C20 heteroaromatic ring.

10. The compound according to any one of claims 1 to 9, **characterized in that** R₃ is selected from the following groups:

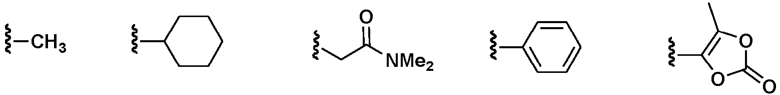

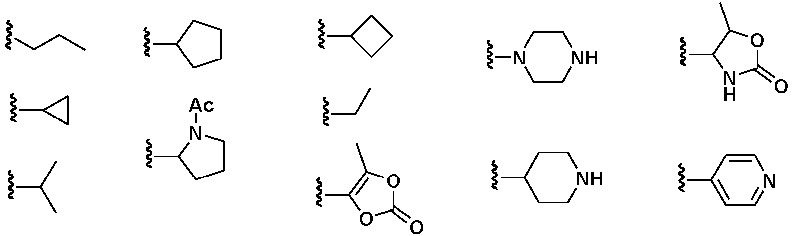

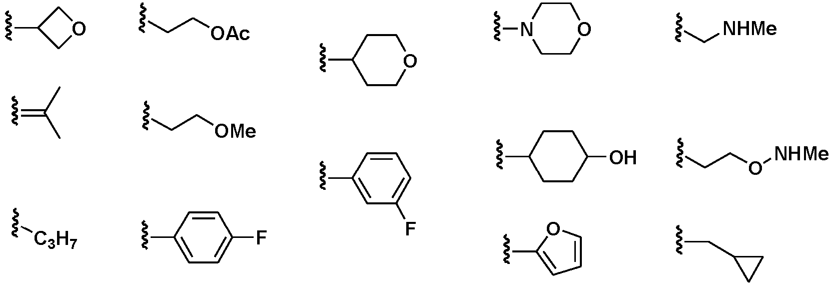

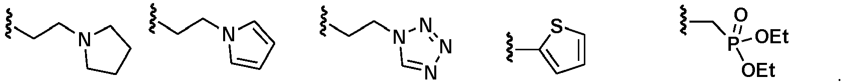

11. The compound according to claim 1, **characterized in that** the compound is selected from the following compounds:

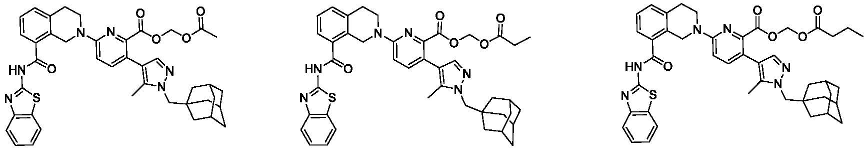

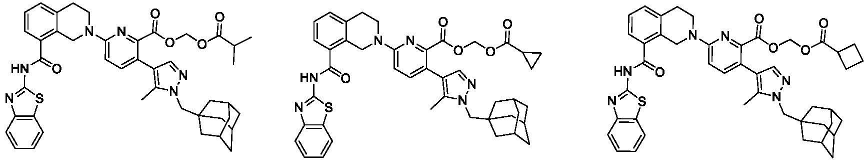

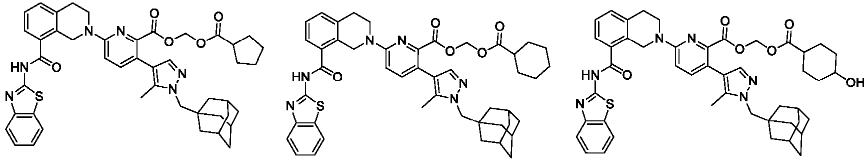

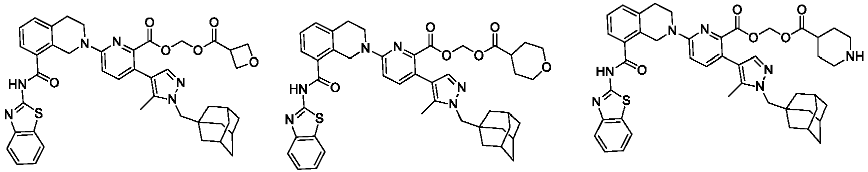

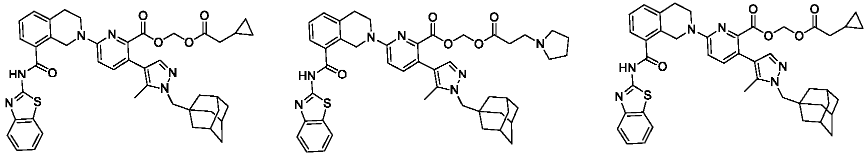

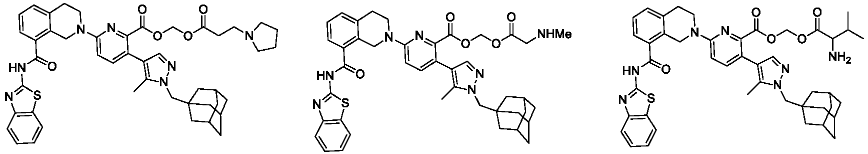

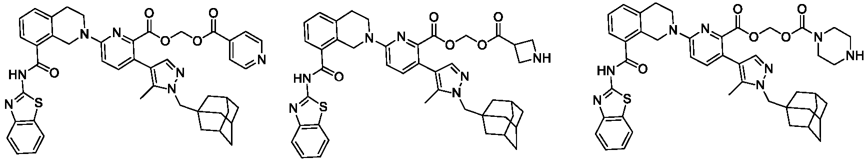

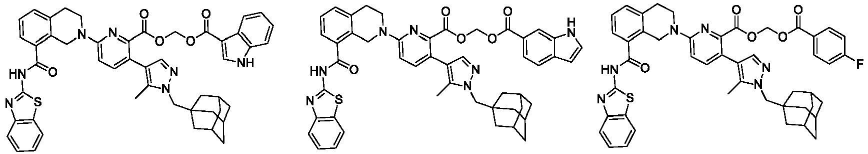

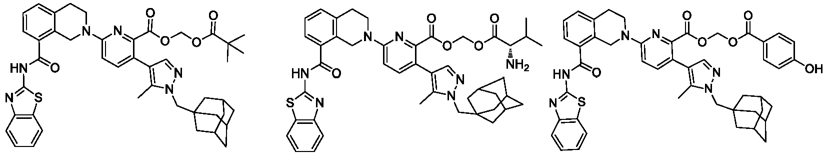

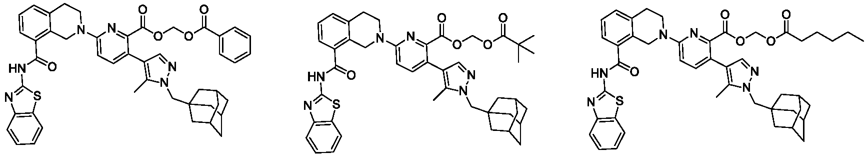

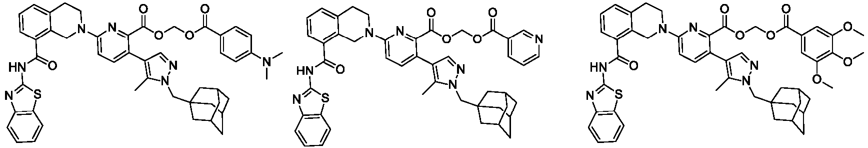

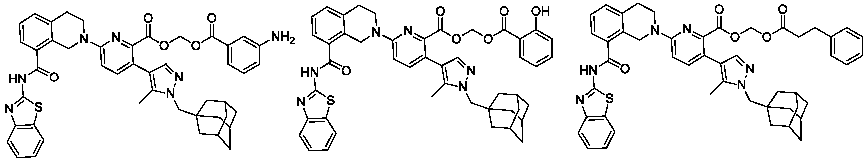

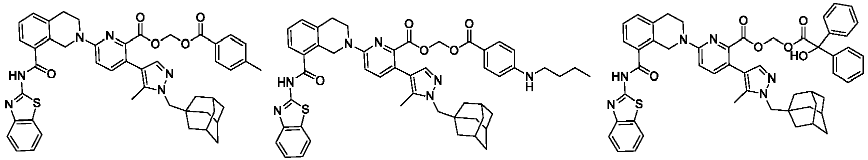

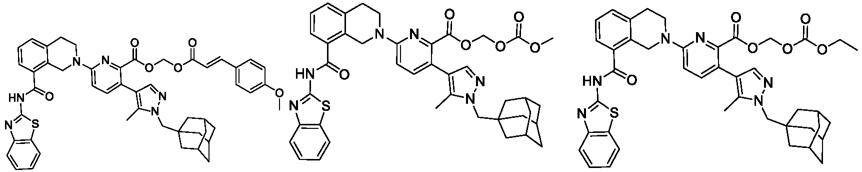

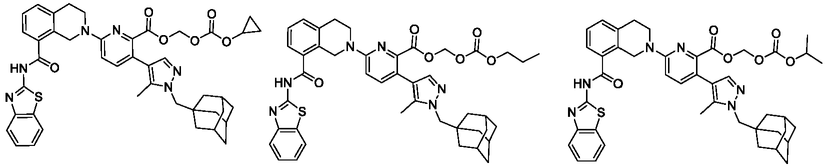

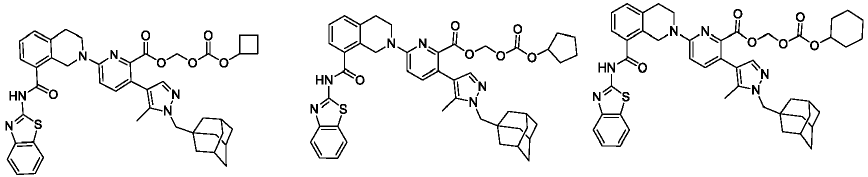

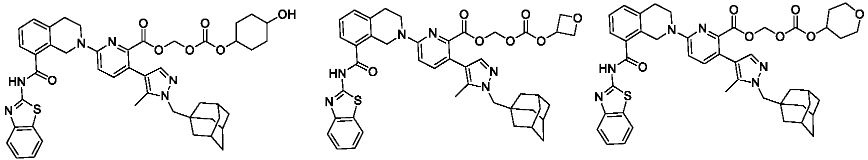

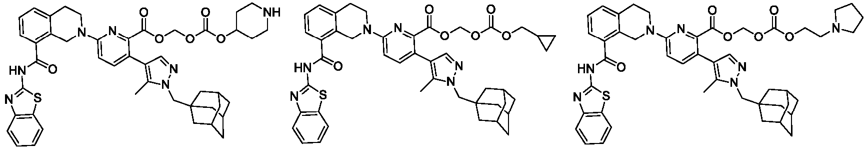

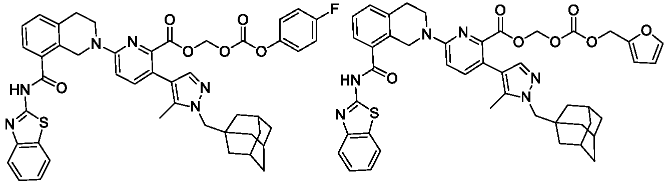

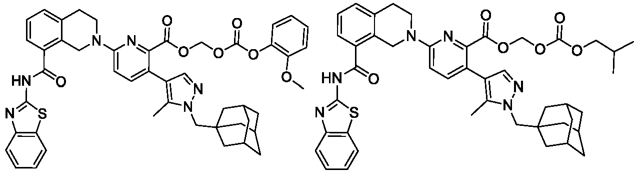

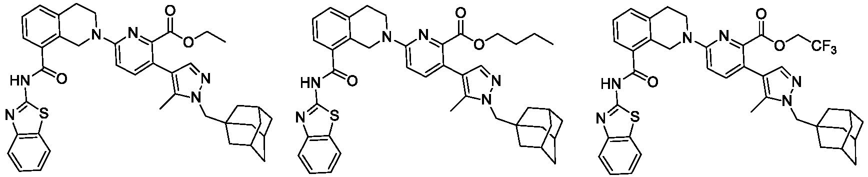

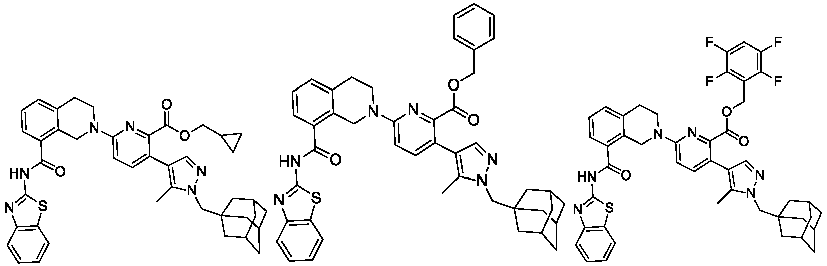

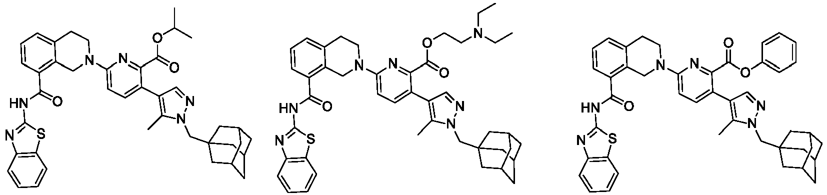

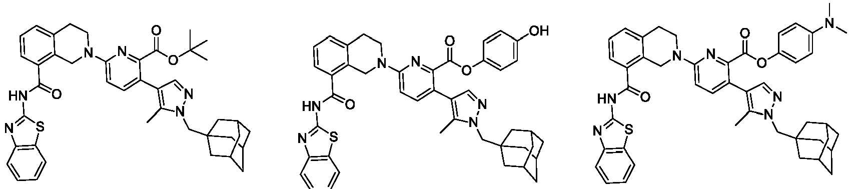

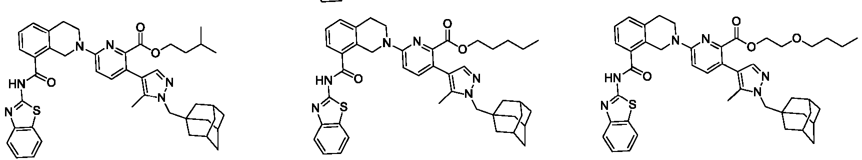

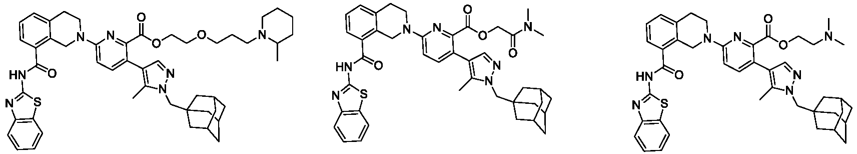

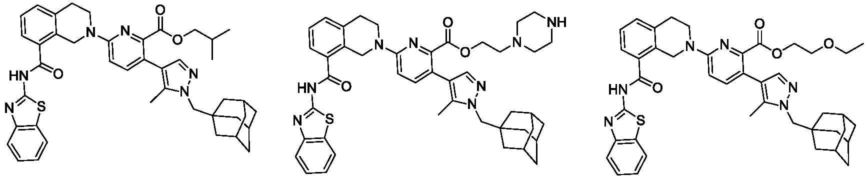

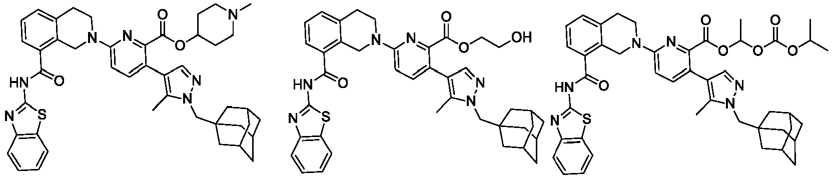

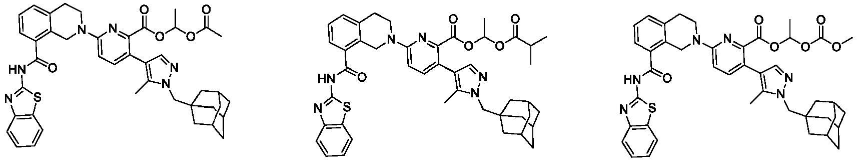

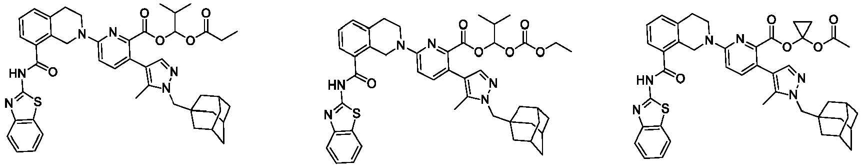

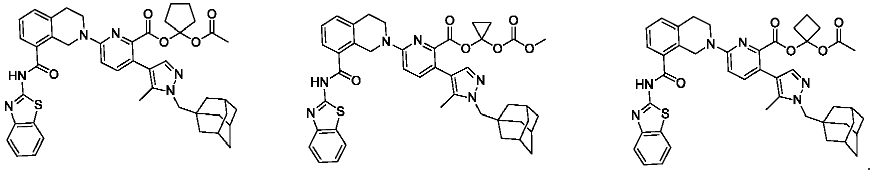

12. A method for preventing or treating aging related diseases, comprising administering to a subject in need a therapeutically effective dosage of the compound according to any one of claims 1 to 10 or a pharmaceutically acceptable salt, solvate, hydrate, polymorph, co-crystal, tautomer, stereoisomer, or isotope labeled compound thereof.

13. The method according to claim 12, **characterized in that** the disease related to aging is a disease related to accumulation of senescent cells, and preferably the disease is one or more selected from the group consisting of idiopathic pulmonary fibrosis, pulmonary fibrosis, hepatic fibrosis, renal fibrosis, inflammation and tissue fibrosis and atrophy of upper respiratory tract and lungs caused by viruses, cystic fibrosis, myelofibrosis, myocardial fibrosis, cutaneous fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritis neuropathy, keratitis sicca, Fuch's corneal dystrophy, presbyopia, cataract, degenerative vitreous disorder including vitreomacular traction syndrome, macular hole, retinal tear, retinal detachment, and proliferative vitreoretinopathy, osteoarthritis, disc herniation, osteoporosis, Alzheimer's disease, Parkinson's disease, atherosclerosis, chronic obstructive pulmonary disease, diabetes, diabetic nephropathy, scar, superficial scar or flat scars, rope scar or contracted scar, webbed scar, depressed scar, atrophic scar, bridge scar and pedunculated scar, hypertrophic scar, keloid, scar cancer, scleroderma, morphea, linear scleroderma, guttate scleroderma, acroscleroderma, diffuse scleroderma, CREST syndrome, acute coronary syndrome, myocardial infarction, stroke, hypertension, obesity, lipodystrophy, coronary artery disease, cerebrovascular disease, periodontal disease, cancer treatment-related disabilities such as atrophy and fibrosis in various tissues, brain and heart damage and treatment-related myelodysplastic syndrome, promyelocytic syndrome, ataxia telangiectasia, Fanconi anemia, Friedreich's ataxia, congenital dyskeratosis, aplastic anemia, aneurysm, inflammatory bowel disease, lipoatrophy, renal transplant failure, sarcopenia, wound healing, alopecia, cardiomyocyte hypertrophy, glomerulosclerosis, and cancer.

14. Use of the compound according to any one of claims 1 to 11 or a pharmaceutically acceptable salt, solvate, hydrate, polymorph, co-crystal, tautomer, stereoisomer, or isotope labeled compound thereof in the preparation of a medicament for preventing or treating diseases related to aging.

15. The use according to claim 14, **characterized in that** the disease related to aging is a disease related to accumulation of senescent cells, and preferably the disease is one or more selected from the group consisting of idiopathic pulmonary fibrosis, pulmonary fibrosis, hepatic fibrosis, renal fibrosis, inflammation and tissue fibrosis and atrophy of upper respiratory tract and lungs caused by viruses, cystic fibrosis, myelofibrosis, myocardial fibrosis, cutaneous fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritis neuropathy, keratitis sicca, Fuch's corneal dystrophy, presbyopia, cataract, degenerative vitreous disorder, including vitreomacular traction syndrome, macular hole, retinal tear, retinal detachment, proliferative vitreoretinopathy, osteoarthritis, disc herniation, osteoporosis, Alzheimer's disease, Parkinson's disease, atherosclerosis, chronic obstructive pulmonary disease, diabetes, diabetic nephropathy, scar, superficial scar or flat scars, rope scar or contracted scar, webbed scar, depressed scar, atrophic scar, bridge scar and pedunculated scar, hypertrophic scar, keloid, scar cancer, scleroderma, morphea, linear scleroderma, guttate scleroderma, acroscleroderma, diffuse scleroderma, CREST syndrome, acute coronary syndrome, myocardial infarction, stroke, hypertension, obesity, lipodystrophy, coronary artery disease, cerebrovascular disease, periodontal disease, cancer treatment-related disabilities such as atrophy and fibrosis in various tissues, brain and heart damage and treatment-related myelodysplastic syndrome, promyelocytic syndrome, ataxia telangiectasia, Fanconi anemia, Friedreich's ataxia, congenital dyskeratosis, aplastic anemia, aneurysm, inflammatory bowel disease, lipoatrophy, renal transplant failure, sarcopenia, wound healing, alopecia, cardiomyocyte hypertrophy, glomerulosclerosis, and cancer.
